# EUROPEAN PATENT APPLICATION

(11) **EP 4 821 947 A1**
(43) Date of publication of application: **14.10.2026**
(21) Application number: 24902676.6
(22) Date of filing: 04.12.2024
(51) Int. Cl.: C07K 16/30, C12N 15/13, C07K 16/46, A61K 39/395, G01N 33/574, G01N 33/68, A61P 35/00

(54) **PSMA ANTIBODY AND USE THEREOF**

(30) Priority: 11.12.2023 CN 202311699412
(71) Applicant: Hefei TG Immunopharma Co., Ltd., Hefei, Anhui 230001 (CN)
(72) Inventor: CAO, Guoshuai, Hefei, Anhui 230001 (CN); CHENG, Ying, Hefei, Anhui 230001 (CN); LI, Yangyang, Hefei, Anhui 230001 (CN); WU, Yuwei, Hefei, Anhui 230001 (CN)
(74) Representative: Potter Clarkson
(86) International application number: PCT/CN2024/136743
(87) International publication number: WO 2025/124255

(57) **Abstract**

Provided are an antibody or antigen-binding fragment targeting PSMA, a bispecific antibody including the antibody or antigen-binding fragment, a nucleic acid molecule encoding the antibody or antigen-binding fragment, an expression vector, a recombinant cell, a conjugate, a pharmaceutical composition, a kit, and uses thereof.

## Description

### PRIORITY INFORMATION

This application claims priority to and benefits of Chinese patent application No. 202311699412.4, filed with China National Intellectual Property Administration on December 11, 2023, the entire contents of which are incorporated herein by reference.

### FIELD

The present disclosure belongs to the technical field of biopharmaceuticals, and specifically, relates to a PSMA antibody and use thereof. More specifically, the present disclosure relates to an antibody or antigen-binding fragment thereof, a bispecific antibody, a nucleic acid molecule, an expression vector, a recombinant cell, a conjugate, a pharmaceutical composition, a kit, and uses thereof.

### BACKGROUND

Cancer is a major disease affecting human survival and development. According to the latest statistics, approximately 19 million new cancer cases and approximately 10 million cancer-related deaths occur worldwide each year, and both the incidence and mortality rates are increasing.

In addition to surgical resection, conventional cancer treatment methods such as chemotherapy and radiotherapy are associated with significant side effects and high recurrence rates. In recent years, immunotherapy, including tumor-targeted antibodies, immune checkpoint antibodies, and bispecific antibodies, has become a new focus and a promising approach against cancer.

In recent years, immunotherapies represented by PD-1/L1 have demonstrated tremendous potential. However, it cannot be ignored that, even for currently approved PD-1/L1 therapies having the broadest indications, the overall response rate remains only about 30%, and many patients still fail to benefit therefrom. Immune checkpoint molecules are inhibitory molecules expressed on the surfaces of immune cells including T cells, NK cells, monocytes, and macrophages. Upon binding to corresponding ligands, these molecules transmit inhibitory signals into immune cells, thereby suppressing the anticancer functions of the immune cells. Due to the substantial heterogeneity in the expression of immune checkpoints affected by their ligands in tumors and tumor-infiltrating lymphocytes, a single type of immune checkpoint therapy cannot be applicable to all patients, and most patients cannot benefit therefrom. On the other hand, some patients receiving immune checkpoint therapy experience tumor recurrence and develop resistance to the immune checkpoint therapy, such that continued administration fails to produce therapeutic efficacy. In addition, T cells recognize neo-antigens, i.e., antigens derived from tumor gene mutations, through T cell receptors (TCRs) on the cell surfaces. Certain tumors, however, exhibit low frequencies of gene mutations and limited types of neo-antigens and are therefore referred to as "cold tumors". Existing immune checkpoint therapies, such as PD-1/L1 therapies, achieve anticancer effects by restoring the intrinsic functions of T cells. However, in "cold tumors," T cells cannot effectively recognize tumor cells, resulting in the ineffectiveness of immune checkpoint therapies against such tumors.

Prostate cancer is one of the most common malignant tumor types in Europe and the United States. With improvements in economic conditions and changes in lifestyle in China, the incidence of prostate cancer in China is also showing an increasing trend. Prostate specific membrane antigen (PSMA) is a cell membrane protein that is scarcely expressed in most normal human tissues but exhibits highly specific overexpression in prostate cancer. More than 80% of prostate cancer patients highly express PSMA, and such high expression is associated with poor prognosis. Therefore, PSMA is a promising therapeutic target, and the development of antibodies targeting PSMA, or bi-/multi-functional antibodies, CAR-T therapies, and CAR-NK therapies based on anti-PSMA monoclonal antibodies, is of significant value.

### SUMMARY

The present disclosure aims to solve at least one of the technical problems existing in the related art, at least to a certain extent. To this end, the present disclosure provides an antibody or antigen-binding fragment targeting PSMA. The antibody or antigen-binding fragment has high binding affinity for PSMA and strong tumor-killing activity.

In a first aspect of the present disclosure, the present disclosure provides an antibody or antigen-binding fragment. According to an embodiment of the present disclosure, the antibody or antigen-binding fragment includes a CDR selected from at least one of: HCDR 1: GYSFTX₁NW, X₁ being S or H; HCDR 2: IYPGDSDT; HCDR 3: ARQTGFLWSSDLWGRGT; LCDR 1: X₂QDISX₃A, X₂ being S or P and X₃ being S or Y; LCDR 2: DASX₄, X₄ being S or W; or LCDR 3: QQFNSYPLX₅, X₅ being T or S, and provided that X₁ being S, X₂ being S, X₃ being S, X₄ being S, and X₅ being T do not occur simultaneously. Compared with a wild-type antibody, the above-described antibody or antigen-binding fragment has high binding affinity for PSMA and strong tumor-killing activity, and is capable of detecting PSMA.

In a second aspect of the present disclosure, the present disclosure provides a multispecific antibody. According to an embodiment of the present disclosure, the multispecific antibody includes: a first binding region including the antibody or antigen-binding fragment in the first aspect, and a second binding region having a binding activity to a first molecule. The multispecific antibody of the present disclosure has high binding affinity for PSMA and strong tumor-killing activity, and is capable of effectively preventing and/or treating a PSMA-mediated associated disease, especially preventing and/or treating prostate cancer.

In a third aspect of the present disclosure, the present disclosure provides a nucleic acid molecule. According to an embodiment of the present disclosure, the nucleic acid molecule encodes the antibody or antigen-binding fragment in the first aspect or the multispecific antibody in the second aspect. The nucleic acid molecule of the present disclosure is capable of encoding the antibody or antigen-binding fragment in the first aspect or the multispecific antibody in the second aspect.

In a fourth aspect of the present disclosure, the present disclosure provides an expression vector. According to an embodiment of the present disclosure, the expression vector carries the nucleic acid molecule in the third aspect. Accordingly, the expression vector of the present disclosure can efficiently achieve the expression of the antibody or antigen-binding fragment in the first aspect or the multispecific antibody in the second aspect, thereby obtaining a large amount of the antibody or antigen-binding fragment or the multispecific antibody *in vitro*.

In a fifth aspect of the present disclosure, the present disclosure provides a recombinant cell. According to an embodiment of the present disclosure, the recombinant cell carries the nucleic acid molecule in the third aspect or the expression vector in the fourth aspect, or the recombinant cell expresses the antibody or antigen-binding fragment in the first aspect or the multispecific antibody in the second aspect. Under suitable conditions, the recombinant cell can effectively express the above-described antibody or antigen-binding fragment or the above-described multispecific antibody.

In a sixth aspect of the present disclosure, the present disclosure provides a conjugate. According to an embodiment of the present disclosure, the conjugate includes: the antibody or antigen-binding fragment in the first aspect or the multispecific antibody in the second aspect, and a conjugate moiety linked to the antibody or antigen-binding fragment or the multispecific antibody. The conjugate of the present disclosure has high binding affinity for PSMA, and is useful in detecting PSMA or preventing and/or treating a PSMA-mediated associated disease, especially in detecting prostate cancer or in preventing and/or treating prostate cancer.

In a seventh aspect of the present disclosure, the present disclosure provides a pharmaceutical composition. According to an embodiment of the present disclosure, the pharmaceutical composition includes the antibody or antigen-binding fragment in the first aspect, the multispecific antibody in the second aspect, the nucleic acid molecule in the third aspect, the expression vector in the fourth aspect, the recombinant cell in the fifth aspect, or the conjugate in the sixth aspect. The pharmaceutical composition of the present disclosure has high binding affinity for PSMA and strong tumor-killing activity, and is capable of effectively preventing and/or treating a PSMA-mediated associated disease, especially preventing and/or treating prostate cancer.

In an eighth aspect of the present disclosure, the present disclosure provides a kit. According to an embodiment of the present disclosure, the kit includes the antibody or antigen-binding fragment in the first aspect, the multispecific antibody in the second aspect, the nucleic acid molecule in the third aspect, the expression vector in the fourth aspect, the recombinant cell in the fifth aspect, or the conjugate in the sixth aspect. The kit of the present disclosure has high binding affinity for PSMA, and is capable of effectively detecting PSMA, detecting a PSMA-mediated associated disease, diagnosing a PSMA-mediated associated disease, staging a PSMA-mediated associated disease, or assessing the prognosis of a PSMA-mediated associated disease.

In a ninth aspect of the present disclosure, the present disclosure provides the antibody or antigen-binding fragment in the first aspect, the multispecific antibody in the second aspect, the nucleic acid molecule in the third aspect, the expression vector in the fourth aspect, the recombinant cell in the fifth aspect, the conjugate in the sixth aspect, or the pharmaceutical composition in the seventh aspect for use in the prevention and/or treatment of a PSMA-mediated associated disease, or their uses in the prevention and/or treatment of a PSMA-mediated associated disease (especially, the prevention and/or treatment of prostate cancer) and/or in the manufacture of a medicament for preventing and/or treating a PSMA-mediated associated disease.

In a tenth aspect of the present disclosure, the present disclosure provides the antibody or antigen-binding fragment in the first aspect, the multispecific antibody in the second aspect, the nucleic acid molecule in the third aspect, the expression vector in the fourth aspect, the recombinant cell in the fifth aspect, the conjugate in the sixth aspect, or the kit in the eighth aspect for use in the detection of PSMA, in the detection of a PSMA-mediated associated disease, in the diagnosis of a PSMA-mediated associated disease, in the staging of a PSMA-mediated associated disease, or in the assessment of the prognosis of a PSMA-mediated associated disease; or their use in: the detection of PSMA, and/or the detection of a PSMA-mediated associated disease, and/or the diagnosis of a PSMA-mediated associated disease, and/or the staging of a PSMA-mediated associated disease, and/or the assessment of the prognosis of a PSMA-mediated associated disease, and/or the manufacture of a kit for detecting PSMA, and/or the manufacture of a kit for detecting a PSMA-mediated associated disease, and/or the manufacture of a kit for diagnosing a PSMA-mediated associated disease, and/or the manufacture of a kit for staging a PSMA-mediated associated disease, and/or the manufacture of a kit for assessing the prognosis of a PSMA-mediated associated disease.

In an eleventh aspect of the present disclosure, the present disclosure provides a method for detecting PSMA. According to an embodiment of the present disclosure, the method includes bring a test sample into contact with the antibody or antigen-binding fragment in the first aspect, the multispecific antibody in the second aspect, or the conjugate in the sixth aspect to form an immune complex. The method of the present disclosure is capable of detecting PSMA and further detecting prostate cancer, especially for *in vitro* detection, and has advantages of high detection accuracy.

In a twelfth aspect of the present disclosure, the present disclosure provides a method for preventing and/or treating a PSMA-mediated associated disease. According to an embodiment of the present disclosure, the method includes administering to a subject a pharmaceutically acceptable amount of the antibody or antigen-binding fragment in the first aspect, the multispecific antibody in the second aspect, the conjugate in the sixth aspect, or the pharmaceutical composition in the seventh aspect. The method of the present disclosure is capable of effectively treating or preventing a tumor and/or cancer, especially preventing and/or treating prostate cancer.

In a thirteenth aspect of the present disclosure, the present disclosure provides a method for diagnosing a PSMA-mediated associated disease. According to an embodiment of the present disclosure, the method includes: detecting PSMA in a test sample using the antibody or antigen-binding fragment in the first aspect, the multispecific antibody in the second aspect, or the conjugate in the sixth aspect; and determining a level of PSMA in the test sample based on a detection result of PSMA. The antibody or antigen-binding fragment provided by the present disclosure, or the antibody or antigen-binding fragment expressed by the nucleic acid molecule, expression vector, or recombinant cell, is each capable of effectively binding to human PSMA. Therefore, the method of the present disclosure is capable of efficiently detecting the level of PSMA in a test sample from a subject and effectively diagnosing a PSMA-mediated associated disease.

In a fourteenth aspect of the present disclosure, the present disclosure provides a method for staging a PSMA-mediated associated disease. According to an embodiment of the present disclosure, the method includes: detecting PSMA in a test sample using the antibody or antigen-binding fragment in the first aspect, the multispecific antibody in the second aspect, or the conjugate in the sixth aspect; and determining a level of PSMA in the test sample based on a detection result of PSMA. The antibody or antigen-binding fragment provided by the present disclosure, or the antibody or antigen-binding fragment expressed by the nucleic acid molecule, expression vector, or recombinant cell, is each capable of effectively binding to human PSMA. Therefore, the method of the present disclosure is capable of efficiently detecting the level of PSMA in a test sample from a subject and evaluating the stage of a PSMA-mediated associated disease based on the level of PSMA.

In a fifteenth aspect of the present disclosure, the present disclosure provides a method for assessing the prognosis of a PSMA-mediated associated disease. According to an embodiment of the present disclosure, the method includes: detecting PSMA in a test sample using the antibody or antigen-binding fragment in the first aspect; and determining a level of PSMA in the test sample based on a detection result of PSMA. As described above, the level of PSMA has a significant impact on cancer. After treatment of an individual suffering from an associated disease, the prognosis of the disease can be effectively evaluated by monitoring the level of PSMA in tissues or excreta of the individual, such as peripheral blood or urine. For example, the prognosis may be evaluated by comparing the level of PSMA in a subject before and after treatment, or by comparing the level of PSMA in a treated subject with the level of PSMA in a normal individual or a diseased individual. The antibody or antigen-binding fragment in the first aspect of the present disclosure, or the antibody or antigen-binding fragment expressed by the nucleic acid molecule, expression vector, or recombinant cell, is each capable of effectively binding to human PSMA. Therefore, the method of the present disclosure is capable of efficiently detecting the level of PSMA in a test sample from a subject and assessing the prognosis of a PSMA-mediated associated disease based on the level of PSMA.

### Beneficial Effects:

1) The affinity-matured PSMA antibody obtained in the present disclosure exhibits a slower dissociation rate (Kd) as compared with the parental antibody.
2) The affinity-matured PSMA antibody obtained in the present disclosure exhibits stronger binding strength to tumor cells as compared with the parental antibody.
3) The affinity-matured PSMA antibody obtained in the present disclosure exhibits higher ADCC activity as compared with the parental antibody.
4) The CD3×PSMA bispecific antibody obtained in the present disclosure exhibits higher binding activity and killing-promoting activity as compared with the parental antibody.

Additional aspects and advantages of the present disclosure will be provided in part in the following description, and in part will become apparent from the following description, or may be learned through practicing of the present disclosure.

### BRIEF DESCRIPTION OF THE DRAWINGS

The above and/or additional aspects and advantages of the present disclosure will become apparent and readily understood from the following description of embodiments taken in conjunction with the accompanying drawings.
FIG. 1 shows a comparison of CDR sequences between the affinity-matured PSMAv11 antibody and the parental mAb according to Example 2 of the present disclosure.
FIG. 2 shows SPR results of protein affinity determination for the affinity-matured PSMAv11 antibody and the parental mAb according to Example 3 of the present disclosure.
FIG. 3 shows flow cytometry results of binding between the affinity-matured PSMAv11 antibody or the parental mAb and 22Rv1 human prostate cancer cells according to Example 5 of the present disclosure.
FIG. 4 shows flow cytometry results of binding between the affinity-matured PSMAv11 antibody or the parental mAb and LNCaP human prostate cancer cells according to Example 5 of the present disclosure.
FIG. 5 shows results of PBMC-mediated killing of LNCaP human prostate cancer cells promoted by the affinity-matured PSMAv11 antibody or the parental mAb according to Example 6 of the present disclosure.
FIG. 6 shows results of PBMC-mediated killing of 22Rv1 human prostate cancer cells promoted by the affinity-matured PSMAv11 antibody or the parental mAb according to Example 6 of the present disclosure.
FIG. 7 shows flow cytometry results of binding between the CD3×PSMAv11 antibody or the CD3×parental PSMA bispecific antibody and 22Rv1 human prostate cancer cells according to Example 7 of the present disclosure.
FIG. 8 shows flow cytometry results of binding between the CD3×PSMAv11 antibody or the CD3×parental PSMA bispecific antibody and LNCaP human prostate cancer cells according to Example 7 of the present disclosure.
FIG. 9 shows results of PBMC-mediated killing of LNCaP human prostate cancer cells promoted by the CD3×PSMAv11 antibody or the CD3×parental PSMA bispecific antibody according to Example 8 of the present disclosure.
FIG. 10 shows results of PBMC-mediated killing of 22Rv1 human prostate cancer cells promoted by the CD3×PSMAv11 antibody or the CD3×parental PSMA bispecific antibody according to Example 8 of the present disclosure.

### DETAILED DESCRIPTION OF THE EMBODIMENTS

Embodiments of the present disclosure will be described in detail below. The embodiments described below are exemplary and are only used to explain the present disclosure, and they should not be construed as limiting the present disclosure.

It should be noted that the terms "first" and "second" are only used for descriptive purposes, rather than indicating or implying relative importance or implicitly indicating the number of indicated technical features. Therefore, the features defined with "first" and "second" may include one or more of these features explicitly or implicitly. Further, in the description of the present disclosure, unless otherwise specified, "more" or "plurality" means two or more.

### Detailed Description of the Present Disclosure

### Definitions and General Terms

As used herein, the term "comprise" or "include" is an open expression, that is, including the contents indicated by the present disclosure, but not excluding other aspects.

As used herein, the terms "optionally" or "optional" generally mean that the event or situation described subsequently may but not necessarily happen, and the description includes the situation in which the event or situation happens and the situation in which the event or situation does not happen.

As used herein, the term "fragment" refers to a target protein or polypeptide, and a target protein or polypeptide with N-terminal end (N-terminus) or C-terminal end (C-terminus) truncation and/or internal deletion.

To make the present disclosure more easily understood, certain technical and scientific terms are specifically defined below. Unless otherwise clearly defined, all other technical and scientific terms used herein have the meanings commonly understood by those skilled in the art to which the present disclosure belongs. The abbreviations for amino acid residues are standard 3-letter and/or 1-letter codes used in the art to refer to one of the 20 commonly used L-amino acids.

The antibody or antigen-binding fragment thereof described in the present disclosure is generally prepared by biosynthesis methods. According to nucleotide sequences in the present disclosure, the encoding nucleic acid according to the present disclosure may be conveniently prepared by those skilled in the art using various known methods. For example, these methods include, but not limited to: polymerase chain reaction (PCR), DNA artificial synthesis, etc. Specific methods may refer to J. Sambrook, Molecular Cloning: Laboratory Manual. As an implementation of the present disclosure, the encoding nucleic acid sequence may be constructed by a method for synthesizing nucleotide sequence fragments followed by overlap extension PCR. The antibody or the antigen fragment is numbered and defined according to the Kabat numbering system.

As used herein, the terms "identity", "homology" or "similarity" are used interchangeably to describe, with respect to an amino acid sequence or a nucleic acid sequence relative to a reference sequence, the percentage of identical amino acids or nucleotides between two amino acid sequences or nucleic acid sequences as determined by conventional methods, for instance, referring to Ausubel, et al., eds. (1995), Current Protocols in Molecular Biology, Chapter 19 (Greene Publishing and Wiley-Interscience, New York); and the ALIGN program described in Dayhoff (1978), Atlas of Protein Sequence and Structure 5: Suppl.3 (National Biomedical Research Foundation, Washington, D.C.). Various algorithms are available for sequence alignment and determination of sequence identity, including the homology alignment algorithm of Needleman et al. (1970) J. Mol. Biol. 48:443; the local homology algorithm of Smith et al. (1981) Adv. Appl. Math. 2:482; the similarity search method of Pearson et al. (1988) Proc. Natl. Acad. Sci. 85:2444; the Smith-Waterman algorithm (Meth. Mol. Biol. 70: 173-187(1997)); and the BLASTP, BLASTN and BLASTX algorithms (referring to Altschul et al. (1990) J. Mol. Biol. 215:403-410). Computer programs implementing these algorithms are also available, and include, but not limited to: ALIGN or Megalign (DNASTAR) software; WU-BLAST-2 (Altschul et al., Meth. Enzym., 266: 460-480(1996)); GAP, BESTFIT, BLAST (Altschul etc., *ibid.*), FASTA, and TFASTA, available in the Genetics Computing Group (GCG) Package, Version 8, Madison, Wisconsin, USA; and the CLUSTAL in the PC/Gene program provided by Intelligenetics, Mountain View, California.

As used herein, the term "at least 80% identity" refers to at least 80% identity to a respective reference sequence, such as 80%, 81%, 82%, 83%, 84%, 85%, 86%, 87%, 88%, 89%, 90%, 91%, 92%, 93%, 94%, 95%, 96%, 97%, 98%, 99%, 99.5%, or 99.9% identity.

As used herein, the term "at least 90% identity" refers to at least 90% identity to a respective reference sequence, such as 90%, 91%, 92%, 93%, 94%, 95%, 96%, 97%, 98%, 99%, 99.5%, or 99.9% identity.

As used herein, the term "expression vector" generally refers to a nucleic acid molecule that can be inserted into a suitable host for self-replication and transfer the inserted nucleic acid molecule into a host cell and/or between host cells. The nucleic acid molecule includes a nucleotide sequence capable of expressing a target protein. The expression vector may include a vector primarily used to insert DNA or RNA into cells, a vector primarily used to replicate DNA or RNA, and a vector primarily used to express, i.e., transcript and/or translate, DNA or RNA. The expression vector also includes vectors with multiple functions as described above. The expression vector may be a polynucleotide capable of being transcribed and translated into a polypeptide when introduced into a suitable host cell. Generally, the expression vector can produce the desired expression product by culturing an appropriate host cell containing the expression vector.

As used herein, the term "recombinant cell" generally refers to a cell with stably inherited unique traits that are obtained by modifying, engineering, or recombining the genetic material of a host cell using genetic engineering technology or cell fusion technology. The term "host cell" refers to a prokaryotic cell or a eukaryotic cell, into which a recombinant expression vector can be introduced. As used herein, the term "transformed" or "transfected" refers to the introduction of a nucleic acid (such as an expression vector) into a cell by various techniques known in the art. Suitable host cells may be transformed or transfected with the DNA sequence of the present disclosure, and may be used for the expression and/or secretion of target proteins. Examples of suitable host cells that can be used in the present disclosure include immortalized hybridoma cells, NS/0 myeloma cells, 293 cells, Chinese hamster ovary (CHO) cells, HeLa cells, Cap cells (cells derived from human amniotic fluid), and CoS cells.

As used herein, the term "pharmaceutical composition" generally refers to a unit dosage form and may be prepared by any method well known in the pharmaceutical field. All methods include the step of combining the active ingredient with a carrier, which constitutes one or more auxiliary ingredients. Generally, the composition is prepared by uniformly and sufficiently combining the active antibody or antigen-binding fragment with a liquid carrier, a finely divided solid carrier, or both.

As used herein, the term "pharmaceutically acceptable excipient" may include any solvent, solid excipient, diluent, or other liquid excipient suitable for a particular intended dosage form. Except to the extent that any conventional excipients are incompatible with the antibody or antigen-binding fragment of the present disclosure, for example, by producing any undesirable biological effects or otherwise interacting in a deleterious manner with any other components of the pharmaceutically acceptable composition, their uses are also contemplated by the present disclosure.

As used herein, the term "administering" or "administration" refers to introducing a predetermined amount of substance into a patient by a certain suitable route. The antibody or antigen-binding fragment, the multispecific antibody, or the pharmaceutical composition of the present disclosure may be administered by any common route, provided that it can reach the intended tissue. Various routes of administration are contemplated, including intraperitoneal administration, intravenous injection, intramuscular injection, subcutaneous injection, etc., although the present disclosure is not limited to these exemplary administration routes. Preferably, the composition of the present disclosure is administered by intravenous injection or subcutaneous injection.

As used herein, the term "treatment" or "treat" refers to obtaining a desired pharmacological and/or physiological effect. The effect may be prophylactic in terms of completely or partially preventing a disease or its symptoms, and/or therapeutic in terms of partially or completely curing a disease and/or adverse effects caused by the disease. As used herein, the term "treatment" or "treat" encompasses treatment of diseases in mammals, especially humans, including: (a) preventing the occurrence of diseases or disorders in individuals who are susceptible to diseases but have not been diagnosed with the diseases; (b) inhibiting diseases, for example, retarding the progression of the diseases; or (c) alleviating diseases, such as alleviating the symptoms associated with the diseases. As used herein, the term "treatment" or "treat" includes treating, curing, alleviating, ameliorating, mitigating or inhibiting a disease in an individual by administrating any of a medicament or an antibody or antigen-binding fragment to the individual, including but not limited to, administering a medicament containing the antibody or antigen-binding fragment described herein to an individual in need thereof.

### Detailed Description of Anti-SARS-CoV-2 Antibody and Use thereof in the Present Disclosure

The present disclosure provides an antibody or antigen-binding fragment, a multispecific antibody, a nucleic acid molecule, an expression vector, a recombinant cell, a conjugate, a pharmaceutical composition, a kit, and uses thereof, which will be described in detail below.

### Antibody or Antigen-binding Fragment

In an aspect of the present disclosure, the present disclosure provides an antibody or antigen-binding fragment. According to an embodiment of the present disclosure, the antibody or antigen-binding fragment includes a CDR selected from at least one of: HCDR 1: GYSFTX₁NW, in which X₁ is S or H; HCDR 2: IYPGDSDT; HCDR 3: ARQTGFLWSSDLWGRGT; LCDR 1: X₂QDISX₃A, in which X₂ is S or P and X₃ is S or Y; LCDR 2: DASX₄, in which X₄ is S or W; or LCDR 3: QQFNSYPLX₅, in which X₅ is T or S, provided that X₁ being S, X₂ being S, X₃ being S, X₄ being S, and X₅ being T do not occur simultaneously. Compared with a wild-type antibody, the above-described antibody or antigen-binding fragment has high binding affinity for PSMA and strong tumor-killing activity, and is capable of detecting PSMA or preventing and/or treating a PSMA-mediated associated disease.

As used herein, the term "antibody" is used in the broadest sense, and it may include full-length monoclonal antibodies, multi-specific antibodies, and chimeric antibodies. The specific structure is not limited as long as they exhibit the desired biological activity. The antibody usually includes a light chain with a lighter molecular weight and a heavy chain with a heavier molecular weight. The heavy chain (H chain) and the light chain (L chain) are linked by disulfide bonds to form an antibody molecule. The amino acid sequence of the amino terminus (N terminus) of the peptide chain varies significantly, which is referred to as a variable region (V region), while the carboxyl terminus (C terminus) is relatively stable and varies insignificantly, which is referred to as a constant region (C region). The V region of the L chain and the V region of the H chain are referred to as VL and VH, respectively.

As used herein, heavy chain complementary determining regions (CDRs of the heavy chain variable region) are represented by "HCDRs" or "HCDR", including HCDR1 (also known as CDR-H1), HCDR2 (also known as CDR-H2), and HCDR3 (also known as CDR-H3). As used herein, light chain complementary determining regions (CDRs of the light chain variable region) are represented by "LCDRs" or "LCDR", including LCDR1 (also known as CDR-L1), LCDR2 (also known as CDR-L2), and LCDR3 (also known as CDR-L3). The definition solutions of CDRs commonly used in the art include: Kabat definition, Chothia definition, IMGT definition, Contact definition, and AbM definition.

As described herein, "Kabat" definition system refers to the definition system described by Kabat et al., U.S. Dept. of Health and Human Services, "Sequence of Proteins of Immunological Interest" (1983). "Chothia" definition system refers to Chothia et al., J Mol Biol 196: 901-917 (1987). As examples, Table A lists the definitions of CDRs. The definitions in different literature may be slightly different. Based on the amino acid sequence of the variable region of a given antibody, those skilled in the art can routinely determine which residues contain a specific CDR. It should be noted that, the CDRs of the present disclosure include, but are not limited to, CDRs defined by a system other than those in Table A, and CDRs determined based on the heavy chain variable region and light chain variable region disclosed in the present disclosure using other rules in the art also fall within the protection scope of the present disclosure.

**[Table A] CDR Definition¹**

| CDR | Kabat | AbM² | IMGT | Chothia |
|---|---|---|---|---|
| HCDR1 | H31~H35³ | H26~H35³ | H26~H33⁵ | H26~H32..34⁴ |
| HCDR2 | H50~H65 | H50~H58 | H51~H57 | H52~H56 |
| HCDR3 | H95~H102 | H95~H102 | H93~H102 | H95~H102 |
| LCDR1 | L24~L34 | L24~L34 | L27~L32 | L24~L34 |
| LCDR2 | L50~L56 | L50~L56 | L50~L51 | L50~L56 |
| LCDR3 | L89~L97 | L89~L97 | L89~L97 | L89~L97 |

| | | | | |
|---|---|---|---|---|
| ¹ The numbering of all CDR definitions in Table A is based on the Kabat numbering system (see below), where the amino acid number on the heavy chain is represented by "H+number", and where the amino acid number on the light chain is represented by "L+number". ² "AbM" used in Table A with a lowercase "b" refers to the CDR defined by "AbM" antibody modeling software of Oxford Molecular. ³ If neither H35A nor H35B exists, CDR-H1 ends at position 35; if only H35A exists, CDR-H1 ends at position 35A; and if both H35A and H35B exist simultaneously, CDR-H1 ends at position 35B. ⁴ If neither H35A nor H35B exists, CDR-H1 ends at position 32; if only H35A exists, CDR-H1 ends at position 33; and if both H35A and H35B exist simultaneously, CDR-H1 ends at position 34. ⁵ If neither H35A nor H35B exists, CDR-H1 ends at position 33; if only H35A exists, CDR-H1 ends at position 34; and if both H35A and H35B exist simultaneously, CDR-H1 ends at position 35. | | | | |

Kabat *et al.* also define a numbering system applicable to the variable region sequences of any antibody. Those skilled in the art can clearly correspond the Kabat numbering system to any variable region sequence without relying on any experimental data other than the sequence itself. As used herein, "Kabat numbering" refers to the numbering using the numbering system described in Kabat et al., U.S. Dept. of Health and Human Services, "Sequence of Proteins of Immunological Interest" (1983)". The HCDRs and LCDRs of the antibody or antigen-binding fragment of the present disclosure are numbered using the above-mentioned numbering system, and specific numbering results are shown in Table A. It should be noted that, the sequence listing and the polypeptide sequences in Table B of the present disclosure are numbered according to the Kabat numbering system. However, those skilled in the art are fully capable of converting the Kabat-numbered sequences in the sequence listing into HCDRs and/or LCDRs under other numbering systems, all of which are within the protection scope of the present disclosure.

As used herein, the term "antigen-binding fragment" refers to a fragment including a portion or all of an antibody, while lacking at least some of the amino acids present in the full-length chain but still capable of specifically binding to an antigen. For example, the fragment may include a portion or all of the CDRs of the antibody. Such fragments are biologically active because they bind to an antigen and can compete with other antigen-binding molecules (including intact antibodies) for binding to a given epitope. Such fragments are selected from Fab, Fv, scFv, or single-domain antibodies. Such fragments may be produced by recombinant nucleic acid technology, or can be produced by enzymatic cleavage or chemical cleavage of antigen-binding molecules (including intact antibodies).

According to an embodiment of the present disclosure, the antibody or antigen-binding fragment may further include at least one of the following additional technical features.

According to an embodiment of the present disclosure, X₁ is S.

According to an embodiment of the present disclosure, X₁ is H.

According to an embodiment of the present disclosure, X₂ is S.

According to an embodiment of the present disclosure, X₂ is P.

According to an embodiment of the present disclosure, X₃ is S.

According to an embodiment of the present disclosure, X₃ is Y.

According to an embodiment of the present disclosure, X₄ is S.

According to an embodiment of the present disclosure, X₄ is W.

According to an embodiment of the present disclosure, X₅ is T.

According to an embodiment of the present disclosure, X₅ is S.

In an embodiment of the present disclosure, the antibody or antigen-binding fragment includes CDRs selected from one of the following groups:

| Group | X₁ | X₂ | X₃ | X₄ | X₅ |
|---|---|---|---|---|---|
| 1 | H | S | S | S | T |
| 2 | S | P | S | S | T |
| 3 | S | S | Y | S | T |
| 4 | S | S | S | W | T |
| 5 | S | S | S | S | S |
| 6 | H | P | S | S | S |
| 7 | H | S | Y | S | T |
| 8 | H | S | S | W | T |
| 9 | H | S | S | S | S |
| 10 | S | P | Y | S | T |
| 11 | S | P | S | W | T |
| 12 | S | P | S | S | S |
| 13 | S | S | Y | W | T |
| 14 | S | S | Y | S | S |
| 15 | S | S | S | W | S |
| 16 | H | P | Y | S | T |
| 17 | H | P | S | W | T |
| 18 | H | P | S | S | S |
| 19 | H | S | Y | W | T |
| 20 | H | S | Y | S | S |
| 21 | H | S | S | W | S |
| 22 | S | P | Y | W | T |
| 23 | S | P | Y | S | S |
| 24 | S | S | Y | W | S |
| 25 | H | P | Y | W | T |
| 26 | H | P | Y | S | S |
| 27 | S | P | Y | W | S |
| 28 | H | P | Y | W | S |

According to an embodiment of the present disclosure, the antibody or antigen-binding fragment includes: HCDR1, HCDR2, and HCDR3 with an amino acid sequence as set forth in SEQ ID NO: 1, SEQ ID NO: 2, and SEQ ID NO: 3, respectively; and LCDR1, LCDR2, and LCDR3 with an amino acid sequence as set forth in SEQ ID NO: 4, SEQ ID NO: 5, and SEQ ID NO: 6, respectively. Such CDRs are identical to those of Group 18 in the above table.

According to an embodiment of the present disclosure, the antibody or antigen-binding fragment thereof specifically recognizes PSMA.

According to an embodiment of the present disclosure, the antibody or antigen-binding fragment includes a heavy chain framework region and/or a light chain framework region.

According to an embodiment of the present disclosure, at least a portion of the heavy chain framework region and/or at least a portion of the light chain framework region is from at least one of a murine antibody, a primate antibody, a bovine antibody, an equine antibody, a dairy cow antibody, a porcine antibody, an ovine antibody, a caprine antibody, a canine antibody, a feline antibody, a rabbit antibody, a camelid antibody, a donkey antibody, a deer antibody, a mink antibody, a chicken antibody, a duck antibody, a goose antibody, a turkey antibody, a gamecock antibody, or a mutant thereof.

According to an embodiment of the present disclosure, at least a portion of the heavy chain framework region and/or at least a portion of the light chain framework region is from at least one of a murine antibody and a human antibody.

According to an embodiment of the present disclosure, the antibody or antigen-binding fragment includes: a heavy chain variable region with an amino acid sequence as set forth in SEQ ID NO: 7 or an amino acid sequence having at least 90% homology thereto, and a light chain variable region with an amino acid sequence as set forth in SEQ ID NO: 8 or an amino acid sequence having at least 90% homology thereto.

According to an embodiment of the present disclosure, the antibody or antigen-binding fragment further includes a heavy chain constant region and/or a light chain constant region.

According to an embodiment of the present disclosure, at least a portion of at least one of the heavy chain constant region and the light chain constant region is from at least one of a murine antibody, a primate antibody, a bovine antibody, an equine antibody, a dairy cow antibody, a porcine antibody, an ovine antibody, a caprine antibody, a canine antibody, a feline antibody, a rabbit antibody, a camelid antibody, a donkey antibody, a deer antibody, a mink antibody, a chicken antibody, a duck antibody, a goose antibody, a turkey antibody, a gamecock antibody, or a mutant thereof.

According to an embodiment of the present disclosure, the heavy chain constant region includes a heavy chain constant region selected from IgG1, IgG2, IgG3, IgG4, IgA, IgM, IgE, or IgD.

According to an embodiment of the present disclosure, the light chain constant region includes a light chain constant region selected from a κ-type light chain constant region or a λ-type light chain constant region.

According to an embodiment of the present disclosure, the light chain constant region and the heavy chain constant region are both from a murine antibody or a mutant thereof, and/or a human antibody or a mutant thereof.

According to an embodiment of the present disclosure, the N-terminus of the heavy chain constant region is linked to the C-terminus of the heavy chain variable region, and/or the N-terminus of the light chain constant region is linked to the C-terminus of the light chain variable region.

According to an embodiment of the present disclosure, the heavy chain constant region includes a heavy chain constant region with an amino acid sequence as set forth in SEQ ID NO: 9 or an amino acid sequence having at least 80% identity thereto, and/or the light chain constant region includes a light chain constant region with an amino acid sequence as set forth in SEQ ID NO: 10 or an amino acid sequence having at least 80% identity thereto.

According to an embodiment of the present disclosure, the antibody includes at least one of a polyclonal antibody, a full-length monoclonal antibody, a Fab antibody, a Fab' antibody, a F(ab')₂ antibody, a Fv antibody, a single-chain antibody, a single-domain antibody, or a minimum recognition unit; or the antigen-binding fragment includes at least one of a F(ab')₂ fragment, a Fab' fragment, a Fab fragment, a F(ab)₂ fragment, a Fv fragment, a scFv fragment, a scFv-Fc fusion protein, a scFv-Fv fusion protein, or a minimum recognition unit.

As used herein, the terms "full-length antibody", "full-length monoclonal antibody", or "full-length monoclonal antibody" are all composed of at least two identical light chains and at least two identical heavy chains linked by interchain disulfide bonds, such as immunoglobulin G (IgG), immunoglobulin A (IgA), immunoglobulin M (IgM), immunoglobulin D (IgD), or immunoglobulin E (IgE).

As used herein, the terms "multispecific antibody" and "multi-antibody" are synonymous, and both refer to an antibody capable of recognizing multiple antigenic epitopes, such as an antibody capable of recognizing two antigenic epitopes (bispecific antibody, abbreviated as BsAb), an antibody capable of recognizing three antigenic epitopes, or an antibody capable of recognizing four antigenic epitopes. The terms are understood in a broad sense and specific structures thereof are not limited, as long as multiple antigenic epitopes can be recognized. In the present disclosure, at least one of the multiple antigenic epitopes is derived from PSMA.

As used herein, the terms "single domain antibody", "nanobody", and "VHH antibody" are used interchangeably. Such antibodies were originally described as the antigen-binding immunoglobulin (variable) domains of "heavy-chain antibodies" (i.e. "antibodies devoid of light chains") (Hamers-Casterman C, AtarhouchT, Muyldermans S, Robinson G, Hamers C, Songa EB, Bendahman N, Hamers R.: "Naturally occurring antibodies devoid of light chains"; Nature 363, 446-448(1993)). These antibodies include a heavy chain variable region (VH) and conventional CH2 and CH3 regions, and specifically binds to an antigenic protein (such as PSMA) through the variable region of the heavy chain.

As used herein, the term "Fab antibody" or "Fab fragment" usually refers to an antibody or fragment containing only Fab molecules. It is composed of VH and CH1 of the heavy chain and the intact light chain, and the light chain and the heavy chain are linked by a disulfide bond.

As used herein, the term "F(ab')₂ antibody" or "F(ab')₂ fragment" has two antigen-binding F(ab') moieties linked together by disulfide bonds.

As used herein, the term "Fv antibody" or "Fv fragment" generally refers to an antibody or a fragment only composed of the light chain variable region (VL) and the heavy chain variable region (VH) linked by non-covalent bonds, and it is the smallest functional fragment of the antibody that retains the complete antigen-binding site.

As used herein, the terms "single-chain antibody" and "scFv fragment" refer to antibodies or fragments composed of the heavy chain variable region and the light chain variable region of the antibody that are linked by a short peptide.

As used herein, the terms "minimum recognition unit" and "MRU" both refer to antibodies or fragments composed of only one CDR, with an extremely small molecular weight that accounts for only about 1% of the complete antibody.

According to an embodiment of the present disclosure, the antibody or antigen-binding fragment includes: a heavy chain with an amino acid sequence as set forth in SEQ ID NO: 11 or an amino acid sequence having at least 80% homology thereto, and a light chain with an amino acid sequence as set forth in SEQ ID NO: 12 or an amino acid sequence having at least 80% homology thereto.

### Multispecific Antibody

In a second aspect of the present disclosure, the present disclosure provides a multispecific antibody. According to an embodiment of the present disclosure, the multispecific antibody includes: a first binding region, including the antibody or antigen-binding fragment in the first aspect, and a second binding region, having a binding activity to a first molecule. The multispecific antibody of the present disclosure has high binding affinity for PSMA and strong tumor-killing activity, and is capable of effectively preventing and/or treating a PSMA-mediated associated disease, especially preventing and/or treating prostate cancer.

According to an embodiment of the present disclosure, the first molecule is selected from at least one of an immune cell surface antigen, a tumor antigen, a virus, a bacterium, an endotoxin, a cytokine, or a cytokine receptor.

As used herein, "immune cell surface antigen" should be understood in a broad sense, and may refer to an immunogenic protein on the surface of immune cells (e.g., T cells, NK cells, and B cells). Examples include, but are not limited to, CD3, BCMA, CTLA-4, LAG-3, TIGIT, CD38, SLAMF7, B7-H3, CD19, CD20, CD30, CD33, CD47, CD52, CD133, RANKL, and CD16a.

As used herein, "tumor antigen" should be understood in a broad sense, and may refer to an immunogenic protein on the surface of tumor cells. Examples include, but are not limited to, PD-L1, PD-1, TGF-β, CEA, GD2, and CD3.

As used herein, "cytokine" should be understood in a broad sense, and may refer to a class of proteins or small molecular polypeptides that can transmit information between cells and have immunomodulatory and effector functions. Examples include, but are not limited to, IL-10, VEGF (including at least one of VEGF-A, VEGF-B, VEGF-C, VEGF-D, VEGF-E, VEGF-F, or PIGF), EpCAM, GM2, and RANKL.

As used herein, "cytokine receptor" should be understood in a broad sense, and may refer to a receptor on the cell surface that can bind to cytokines. Examples include, but are not limited to, Her2, EGFR and IL-10R.

According to an embodiment of the present disclosure, the first molecule is selected from at least one of CD3, PD-L1, PD-1, IL-10, IL-10R, BCMA, VEGF, TGF-β, CTLA-4, LAG-3, TIGIT, CEA, CD38, SLAMF7, B7-H3, Her2, EpCAM, CD19, CD20, CD30, CD33, CD47, CD52, CD133, EGFR, GD2, CD3, GM2, RANKL, and CD16a.

According to an embodiment of the present disclosure, the second binding region is a binding protein or fragment thereof of the first molecule.

According to an embodiment of the present disclosure, the second binding region is at least one of an antibody or antigen-binding fragment of the first molecule and a receptor fragment of the first molecule.

According to an embodiment of the present disclosure, the second binding region is a single-chain antibody of the first molecule.

According to an embodiment of the present disclosure, the second binding region further includes a first Fc fragment.

According to an embodiment of the present disclosure, the first binding region further includes a second Fc fragment.

Unless otherwise specified herein, the Fc fragment includes CH2 and CH3 regions and optionally a hinge region, such as the first Fc fragment and/or the second Fc fragment. In an embodiment of the present disclosure, the C-terminus of the CH2 region is linked to the N-terminus of the CH3 region. In another embodiment of the present disclosure, the C-terminus of the hinge region is linked to the N-terminus of the CH2 region, and the C-terminus of the CH2 region is linked to the N-terminus of the CH3 region.

According to an embodiment of the present disclosure, both the first Fc fragment and the second Fc fragment are human Fc peptide segments.

According to an embodiment of the present disclosure, the human Fc peptide segment is a human IgG1 Fc peptide segment.

According to an embodiment of the present disclosure, the first Fc fragment and the second Fc fragment are linked via a knock-into-hole structure.

In an embodiment of the present disclosure, the term "knob-into-hole structure" refers to mutations introduced into the CH3 domains of antibody heavy chain constant regions to form "knob" and "hole" mutations, thereby facilitating heavy chain pairing and formation of heterodimers. For example, such a structure may be achieved by introducing mutations into amino acids in the CH3 domains of human IgG1 heavy chain constant regions, where one chain includes T366S, L368A, Y407V, and Y349C mutations (i.e., the "hole"), and the other chain includes T366W and S354C mutations (i.e., the "knob").

According to an embodiment of the present disclosure, the C-terminus of the binding protein or fragment thereof of the first molecule is linked to the N-terminus of the first Fc fragment.

It should be noted that, when the binding protein or fragment thereof of the first molecule is an antibody containing two chains, a peptide chain containing a heavy chain is preferably linked to the first Fc fragment.

According to an embodiment of the present disclosure, the second binding region further includes a linking peptide.

According to an embodiment of the present disclosure, the C-terminus of the binding protein or fragment thereof of the first molecule is linked to the N-terminus of the linking peptide, and the C-terminus of the linking peptide is linked to the N-terminus of the first Fc fragment.

According to an embodiment of the present disclosure, the linking peptide has an amino acid sequence as set forth in (GGGGS)ₙ, where n is an integer greater than or equal to 1, preferably 1, 2, 3, 4, 5, 6, 7, 8, 9, or 10.

According to an embodiment of the present disclosure, the bispecific antibody includes a symmetric bispecific antibody or an asymmetric bispecific antibody, preferably an asymmetric bispecific antibody.

According to an embodiment of the present disclosure, the first molecule is CD3, and the second binding region includes an anti-CD3 antibody.

According to an embodiment of the present disclosure, the anti-CD3 antibody is selected from at least one of a Fab antibody, a Fab' antibody, a F(ab')₂ antibody, a Fv antibody, a single-chain antibody, a single-domain antibody, or a minimum recognition unit.

According to an embodiment of the present disclosure, the anti-CD3 antibody is a CD3 single-chain antibody.

According to an embodiment of the present disclosure, the C-terminus of the CD3 single-chain antibody is linked to the N-terminus of the linking peptide, and the C-terminus of the linking peptide is linked to the N-terminus of the first Fc fragment.

According to an embodiment of the present disclosure, the CD3 single-chain antibody has an amino acid sequence as set forth in SEQ ID NO: 13.

According to an embodiment of the present disclosure, the linking peptide has an amino acid sequence as set forth in SEQ ID NO: 14.

According to an embodiment of the present disclosure, the first Fc fragment has an amino acid sequence as set forth in SEQ ID NO: 15.

According to an embodiment of the present disclosure, the first binding region has an amino acid sequence as set forth in SEQ ID NO: 17.

According to an embodiment of the present disclosure, the antibody or antigen-binding fragment in the first binding region is selected from at least one of a Fab antibody, a Fab' antibody, an F(ab')₂ antibody, a Fv antibody, a single-chain antibody, a single-domain antibody, or a minimum recognition unit, preferably a Fab antibody or a single-chain antibody

According to an embodiment of the present disclosure, the antibody or antigen-binding fragment in the first binding region is a Fab antibody.

According to an embodiment of the present disclosure, the Fab antibody in the first binding region includes CDRs of the antibody or antigen-binding fragment in the first aspect.

As defined above, the Fab antibody includes two chains, that is, a heavy chain variable region + CH1 and a light chain variable region + CL. The CDRs in the heavy chain variable region and in the light chain variable region are consistent with the CDRs defined in the antibody or antigen-binding fragment in the first aspect.

According to an embodiment of the present disclosure, the Fab antibody in the first binding region includes the heavy chain variable region and the light chain variable region of the antibody or antigen-binding fragment in the first aspect.

As defined above, the Fab antibody includes two chains, that is, a heavy chain variable region + CH1 and a light chain variable region + CL (i.e., the light chain constant region). The heavy chain variable region and the light chain variable region are consistent with the heavy chain variable region and the light chain variable region defined in the antibody or antigen-binding fragment in the first aspect.

According to an embodiment of the present disclosure, CH1 in the first binding region has an amino acid sequence as set forth in SEQ ID NO: 22.

According to an embodiment of the present disclosure, the C-terminus of CH1 in the Fab antibody of the first binding region is linked to the N-terminus of the second Fc fragment.

According to an embodiment of the present disclosure, the second Fc fragment has an amino acid sequence as set forth in SEQ ID NO: 16,

According to an embodiment of the present disclosure, the first binding region includes: a first peptide chain having an amino acid sequence as set forth in SEQ ID NO: 18, and a second peptide chain having an amino acid sequence as set forth in SEQ ID NO: 12.

According to an embodiment of the present disclosure, the bispecific antibody includes: a first peptide chain having an amino acid sequence as set forth in SEQ ID NO: 18, a second peptide chain having an amino acid sequence as set forth in SEQ ID NO: 12, and a third peptide chain having an amino acid sequence as set forth in SEQ ID NO: 17.

### Nucleic Acid Molecule, Expression Vector, and Recombinant Cell

In a third aspect of the present disclosure, the present disclosure provides a nucleic acid molecule. According to an embodiment of the present disclosure, the nucleic acid molecule encodes the antibody or antigen-binding fragment in the first aspect or the multispecific antibody in the second aspect. The nucleic acid molecule of the present disclosure is capable of encoding the antibody or antigen-binding fragment in the first aspect or the multispecific antibody in the second aspect.

According to an embodiment of the present disclosure, the nucleic acid molecule is DNA.

It should be noted that, with respect to the nucleic acid molecules mentioned herein, those skilled in the art should understand that they actually include either strand or both strands of a complementary double-stranded nucleic acid. In this specification and claims, for convenience, although only one strand is provided in most cases, the other strand complementary to the one strand is actually also disclosed. In addition, the nucleic acid sequences in the present disclosure include a DNA form or an RNA form, the disclosure of one form means that the other form is also disclosed.

In a fourth aspect of the present disclosure, the present disclosure provides an expression vector. According to an embodiment of the present disclosure, the expression vector carries the nucleic acid molecule in the third aspect. When the above-described nucleic acid molecule is linked to the vector, the nucleic acid molecule may be directly or indirectly linked to control elements on the expression vector, as long as such control elements can control the translation, expression and the like of the nucleic acid molecule. Of course, such control elements may be directly derived from the expression vector itself or may be exogenous, i.e., not derived from the expression vector itself. The nucleic acid molecule only needs to be operably linked to the control elements.

As used herein, the term "operably linked" refers to linking an exogenous gene to an expression vector, such that the control elements in the expression vector, for example, transcription control sequences and translation control sequences, are capable of performing their intended functions in regulating transcription and translation of the exogenous gene. Commonly used expression vectors may be, for example, plasmids, bacteriophages, and the like.

According to some specific embodiments of the present disclosure, after the expression vector is introduced into a suitable recipient cell, the expression of the above-described antibody or the antigen-binding fragment, the above-described recombinant protein, or the above-described multispecific antibody can be effectively achieved under the mediation of a regulatory system, thereby enabling large-scale *in vitro* production of the antibody or antigen-binding fragment, recombinant protein, or multispecific antibody.

According to an embodiment of the present disclosure, the expression vector is selected from a eukaryotic expression vector or a prokaryotic expression vector.

In an optional embodiment of the present disclosure, the expression vector is a plasmid expression vector or a viral expression vector, such as a lentiviral expression vector.

In a fifth aspect of the present disclosure, the present disclosure provides a recombinant cell. According to an embodiment of the present disclosure, the recombinant cell carries the nucleic acid molecule in the third aspect or the expression vector in the fourth aspect, or the recombinant cell expresses the antibody or antigen-binding fragment in the first aspect or the multispecific antibody in the second aspect. Under suitable conditions, the recombinant cell can effectively express the above-described antibody or antigen-binding fragment or the above-described multispecific antibody in the cell.

According to some specific embodiments of the present disclosure, the recombinant cell can efficiently and abundantly express the antibody or antigen-binding fragment thereof under suitable conditions. The antibody or antigen-binding fragment thereof has stronger specificity, a longer half-life, and higher efficacy, and can deliver an antibody medicament to target cells at a lower dosage, thereby effectively treating or preventing a PSMA-mediated disease with reduced toxic and side effects and higher safety.

It should be noted that the term "suitable conditions" refers to conditions suitable for the expression of the antibody or antigen-binding fragment, the recombinant protein, or the multispecific antibody described in the present disclosure. Those skilled in the art would readily understand that the conditions suitable for the expression of the antibody or antigen-binding fragment, the recombinant protein, or the multispecific antibody include, but are not limited to, appropriate transformation or transfection methods, appropriate transformation or transfection conditions, healthy host cell states, appropriate host cell density, suitable cell culture environment, and suitable cell culture time. The "suitable conditions" are not particularly limited, and those skilled in the art may optimize the most suitable conditions for the expression of the antibody or antigen-binding fragment, the recombinant protein, or the multispecific antibody according to specific environments of the laboratory.

According to an embodiment of the present disclosure, the recombinant cell is obtained by introducing the expression vector in the fourth aspect into a host cell.

It should be noted that, the recombinant cell according to the present disclosure is not specially limited and may be a prokaryotic cell, a eukaryotic cell, or a bacteriophage. The prokaryotic cell may be *Escherichia coli*, *Bacillus subtilis*, *Streptomyces*, or *Proteus mirabilis*. The eukaryotic cell includes: fungi such as *Pichia pastoris*, *Saccharomyces cerevisiae*, *Schizosaccharomyces*, and *Trichoderma*; insect cells such as *Spodoptera frugiperda* cells; plant cells such as tobacco cells; and mammalian cells such as BHK cells, CHO cells, COS cells, and myeloma cells. In some embodiments, the recombinant cell according to the present disclosure is preferably a mammalian cell, including BHK cells, CHO cells, NSO cells, or COS cells, and excluding animal germ cells, fertilized eggs, or embryonic stem cells.

According to an embodiment of the present disclosure, the recombinant cell is a eukaryotic cell, preferably a mammalian cell.

### Conjugate, Pharmaceutical Composition, and Kit

In a sixth aspect of the present disclosure, the present disclosure provides a conjugate. According to an embodiment of the present disclosure, the conjugate includes: the antibody or antigen-binding fragment in the first aspect or the multispecific antibody in the second aspect, and a conjugate moiety, linked to the antibody or antigen-binding fragment or the multispecific antibody. The conjugate of the present disclosure has high binding affinity for PSMA, and is useful in detecting PSMA or preventing and/or treating a PSMA-mediated associated disease, especially in detecting prostate cancer or in preventing and/or treating prostate cancer.

According to an embodiment of the present disclosure, the conjugate moiety includes at least one selected from a carrier, a drug, a toxin, a cytokine, a protein tag, or a modifier.

As used herein, the carrier may be a substance that can be suspended or dispersed in a liquid phase (e.g., a solid-phase carrier such as particles or magnetic beads), or a solid phase that can contain or support a liquid phase (e.g., a support such as a plate, a membrane, and a test tube, as well as a container such as a well plate, a microfluidic channel, a glass capillary, a nanocolumn, and a monolithic column). The carrier may also be a labeling carrier used to label an antibody or antigen-binding fragment, a recombinant protein, or a multispecific antibody, such as an enzyme (e.g., peroxidase, alkaline phosphatase, luciferin, and β-galactosidase), an affinity substance (e.g., one of streptavidin and biotin, or one of complementary sense and antisense nucleic acid strands), a fluorescent substance (e.g., fluorescein, fluorescein isothiocyanate, rhodamine, green fluorescent protein, and red fluorescent protein), a luminescent substance (e.g., luciferin, aequorin, acridinium ester, tris(2,2'-bipyridine)ruthenium, and luminol), a radioactive isotope (e.g., ³H, ¹⁴C, ³²P, ³⁵S, and ¹²⁵I), and colloidal gold.

According to an embodiment of the present disclosure, the drug is a small-molecule drug that can bind to the antibody or antigen-binding fragment, the recombinant protein, or the multispecific antibody.

According to an embodiment of the present disclosure, the protein tag includes, but is not limited to, a His tag, Flag tag, GST tag, MBP tag, SUMO tag, and C-Myc tag.

According to an embodiment of the present disclosure, the modifier should be understood in a broad sense and may refer to a substance used to modify a protein. Exemplarily, it may be polyethylene glycol or a derivative thereof.

It should be noted that methods for conjugating the conjugate moiety with the antibody or antigen-binding fragment, the recombinant protein, or the multispecific antibody may employ methods known in the art. Examples include physical adsorption methods, covalent coupling methods, methods utilizing affinity substances (e.g., biotin and streptavidin), and ionic coupling methods.

In a seventh aspect of the present disclosure, the present disclosure provides a pharmaceutical composition. According to an embodiment of the present disclosure, the pharmaceutical composition includes: the antibody or antigen-binding fragment in the first aspect, the multispecific antibody in the second aspect, the nucleic acid molecule in the third aspect, the expression vector in the fourth aspect, the recombinant cell in the fifth aspect, or the conjugate in the sixth aspect. The pharmaceutical composition of the present disclosure has high binding affinity for PSMA and strong tumor-killing activity, and is capable of effectively preventing and/or treating a PSMA-mediated associated disease, especially preventing and/or treating prostate cancer.

According to an embodiment of the present disclosure, the pharmaceutical composition further includes a pharmaceutically acceptable excipient.

The administration of the pharmaceutical composition of the present disclosure may be carried out by any acceptable mode of administration. The pharmaceutical composition of the present disclosure may be formulated into formulations in solid, semi-solid, liquid, or gaseous forms, such as injections and lyophilized powders. Current methods for preparing such dosage forms are known or apparent to those skilled in the art. Typical routes for administering such pharmaceutical compositions include, but are not limited to subcutaneous, intravenous, intramuscular, intradermal, or intrasternal injection, or infusion techniques. The pharmaceutical composition of the present disclosure is formulated to allow the biologically active ingredients contained therein to be bioavailable after the composition is administered to a patient.

In an eighth aspect of the present disclosure, the present disclosure provides a kit. According to an embodiment of the present disclosure, the kit includes: the antibody or antigen-binding fragment in the first aspect, the multispecific antibody in the second aspect, the nucleic acid molecule in the third aspect, the expression vector in the fourth aspect, the recombinant cell in the fifth aspect, or the conjugate in the sixth aspect. The kit of the present disclosure has high binding affinity for PSMA, and is capable of effectively detecting PSMA and further diagnosing a tumor or cancer (especially prostate cancer).

As mentioned above, the antibody or antigen-binding fragment according to some specific embodiments of the present disclosure is capable of effectively binding to human PSMA protein. Therefore, the kit including the antibody or antigen-binding fragment is capable of effectively performing qualitative or quantitative detection of human PSMA protein. The kit provided by the present disclosure may be used, for example, in immunoblotting, immunoprecipitation, and other kits involving detection utilizing the specific binding between human PSMA and the antibody. Such kits may include any one or more of the following: antagonists, anti-PSMA antibodies, or pharmaceutical reference materials; protein purification columns; immunoglobulin affinity purification buffering agents; cell assay diluents; manuals or literatures; and the like. The anti-PSMA antibody may be used in different types of diagnostic tests, for example, for *in vitro* or *in vivo* detecting the presence of various diseases, drugs, toxins or other proteins. For example, an associated disease may be tested by detecting serum or blood from a subject, or scientific research may be conducted to detect human PSMA protein in a test sample. The associated disease may include PSMA related diseases, such as cancer. Certainly, the antibody or antigen-binding fragment thereof provided in the present disclosure may also be used for radioimmunoassay and radioimmunotherapy for the above diseases. The above-mentioned binding molecules are likewise applicable to the above application scenarios and will not be described repeatedly herein.

According to some specific embodiments of the present disclosure, the kit may further include conventional reagents used for detecting PSMA, such as a coating solution, and the like.

### Use

In a ninth aspect of the present disclosure, the present disclosure provides the antibody or antigen-binding fragment in the first aspect, the multispecific antibody in the second aspect, the nucleic acid molecule in the third aspect, the expression vector in the fourth aspect, the recombinant cell in the fifth aspect, the conjugate in the sixth aspect, or the pharmaceutical composition in the seventh aspect for use in the prevention and/or treatment of a PSMA-mediated associated disease, or their use in: the prevention and/or treatment of a PSMA-mediated associated disease (especially, the prevention and/or treatment of prostate cancer), and/or the manufacture of a medicament for preventing and/or treating a PSMA-mediated associated disease.

According to an embodiment of the present disclosure, the PSMA-mediated associated disease includes a tumor or cancer.

According to an embodiment of the present disclosure, the cancer is at least one of prostate cancer, lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head and neck cancer.

According to an embodiment of the present disclosure, the cancer is prostate cancer.

In a tenth aspect of the present disclosure, the present disclosure provides the antibody or antigen-binding fragment in the first aspect, the multispecific antibody in the second aspect, the nucleic acid molecule in the third aspect, the expression vector in the fourth aspect, the recombinant cell in the fifth aspect, the conjugate in the sixth aspect, or the kit in the eighth aspect for use in the detection of PSMA, in the detection of a PSMA-mediated associated disease, in the diagnosis of a PSMA-mediated associated disease, in the staging of a PSMA-mediated associated disease, or in the assessment of the prognosis of a PSMA-mediated associated disease; or their use in: the detection of PSMA, and/or the detection of a PSMA-mediated associated disease, and/or the diagnosis of a PSMA-mediated associated disease, and/or the staging of a PSMA-mediated associated disease, and/or the assessment of the prognosis of a PSMA-mediated associated disease, and/or the manufacture of a kit for detecting PSMA, and/or the manufacture of a kit for detecting a PSMA-mediated associated disease, and/or the manufacture of a kit for diagnosing a PSMA-mediated associated disease, and/or the manufacture of a kit for staging a PSMA-mediated associated disease, and/or the manufacture of a kit for assessing the prognosis of a PSMA-mediated associated disease.

According to an embodiment of the present disclosure, the PSMA-mediated associated disease includes a tumor and/or cancer.

According to an embodiment of the present disclosure, the cancer is at least one of prostate cancer, lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head and neck cancer.

According to an embodiment of the present disclosure, the cancer is prostate cancer.

### Method

In an eleventh aspect of the present disclosure, the present disclosure provides a method for detecting PSMA. According to an embodiment of the present disclosure, the method includes bring a test sample into contact with the antibody or antigen-binding fragment in the first aspect, the multispecific antibody in the second aspect, or the conjugate in the sixth aspect to form an immune complex. The method of the present disclosure is capable of detecting PSMA and further detecting prostate cancer, especially for *in vitro* detection, and has advantages of high detection accuracy.

According to an embodiment of the present disclosure, the presence of SARS-CoV-2 or its level in the test sample is determined based on the signal of the immune complex.

According to an embodiment of the present disclosure, the immune complex further includes a second antibody binding to the antibody or antigen-binding fragment.

According to an embodiment of the present disclosure, the immune complex further includes a second antibody binding to SARS-CoV-2.

In a twelfth aspect of the present disclosure, the present disclosure provides a method for preventing and/or treating a PSMA-mediated associated disease. According to an embodiment of the present disclosure, the method includes administering to a subject a pharmaceutically acceptable amount of the antibody or antigen-binding fragment in the first aspect, the multispecific antibody in the second aspect, the conjugate in the sixth aspect, or the pharmaceutical composition in the seventh aspect. The method of the present disclosure is capable of effectively treating or preventing a tumor and/or cancer, especially preventing and/or treating prostate cancer.

The effective amount of the antibody or antigen-binding fragment, the multispecific antibody, the pharmaceutical composition, or the conjugate of the present disclosure may vary depending on factors such as the mode of administration or the severity of the disease to be treated. The selection of a preferred effective amount may be determined by those of ordinary skill in the art based on various factors (e.g., through clinical trials). Such factors include, but are not limited to: pharmacokinetic parameters of the active ingredient, such as bioavailability, metabolism, and half-life; the severity of the disease to be treated in the patient; the patient's body weight; the immune status of the patient, and the route of administration. For example, depending on exigencies of the therapeutic situation, several divided doses may be administered daily, or the dosage may be proportionally reduced.

The antibody or antigen-binding fragment, the multispecific antibody, the pharmaceutical composition, or the conjugate of the present disclosure may be incorporated into drugs suitable for parenteral administration (e.g., intravenous, subcutaneous, intraperitoneal, or intramuscular administration). These drugs may be prepared in various forms, such as liquid, semi-solid, and solid dosage forms, including but not limited to liquid solutions (e.g., injection solutions and infusion solutions) or lyophilized powders. Typical drugs are in the form of injection solutions or infusion solutions. The above-described antibody or antigen-binding fragment, multispecific antibody, pharmaceutical composition, or conjugate can be administered by intravenous infusion or injection, or by intramuscular or subcutaneous injection.

As used herein, the term "subject" refers to a vertebrate, preferably a mammal, and most preferably a human. Mammals include, but are not limited to, rodents, primates, humans, livestock, sporting animals, and pets. Tissues, cells and progeny thereof from biological entities obtained *in vivo* or cultured *in vitro* are also included.

According to an embodiment of the present disclosure, the route of administration of the method is subcutaneous injection or intravenous injection.

According to an embodiment of the present disclosure, the cancer is at least one of prostate cancer, lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head and neck cancer.

In a thirteenth aspect of the present disclosure, the present disclosure provides a method for diagnosing a PSMA-mediated associated disease. According to an embodiment of the present disclosure, the method includes: detecting PSMA in a test sample using the antibody or antigen-binding fragment in the first aspect, the multispecific antibody in the second aspect, or the conjugate in the sixth aspect; and determining a level of PSMA in the test sample based on a detection result of PSMA. The antibody or antigen-binding fragment provided by the present disclosure, or the antibody or antigen-binding fragment expressed by the nucleic acid molecule, expression vector, or recombinant cell, is each capable of effectively binding to human PSMA protein. Therefore, the method of the present disclosure is capable of efficiently detecting the level of PSMA in a test sample from a subject and effectively diagnosing a PSMA-mediated associated disease.

According to an embodiment of the present disclosure, the method for diagnosing the disease may further include at least one of the following additional technical features.

According to an embodiment of the present disclosure, the level of PSMA in the test sample that is not lower than the minimum standard associated with disease is indicative that the sample is from a patient suffering from a PSMA-mediated associated disease. The value of the minimum standard may be determined through differential comparative analysis and validation of the levels of PSMA in test samples obtained from a large number of individuals suffering from the PSMA-mediated associated disease and a large number of healthy individuals.

According to an embodiment of the present disclosure, the test sample includes at least one of blood, saliva, sweat, tissues, cells, blood, serum, plasma, feces, and urine.

According to an embodiment of the present disclosure, the PSMA-mediated associated disease includes cancer.

According to an embodiment of the present disclosure, the cancer is at least one of prostate cancer, lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head and neck cancer.

In a fourteenth aspect of the present disclosure, the present disclosure provides a method for staging a PSMA-mediated associated disease. According to an embodiment of the present disclosure, the method includes: detecting PSMA in a test sample using the antibody or antigen-binding fragment in the first aspect, the multispecific antibody in the second aspect, or the conjugate in the sixth aspect; and determining a level of PSMA in the test sample based on a detection result of PSMA. The antibody or antigen-binding fragment provided by the present disclosure, or the antibody or antigen-binding fragment expressed by the nucleic acid molecule, expression vector, or recombinant cell, is each capable of effectively binding to human PSMA. Therefore, the method of the present disclosure is capable of efficiently detecting the level of PSMA in a test sample from a subject and evaluating the stage of a PSMA-mediated associated disease based on the level of PSMA.

According to an embodiment of the present disclosure, the method for staging the disease may further include at least one of the following additional technical features.

According to an embodiment of the present disclosure, the level of PSMA in the test sample that is not lower than the standard level associated with stage IV tumor disease is indicative that the test sample is from a patient suffering from stage IV tumor disease; the level of PSMA in the test sample that falls between the standard levels associated with stage IV and stage III tumor disease is indicative that the test sample is from a patient suffering from stage III tumor disease; the level of PSMA in the test sample that falls between the standard levels associated with stage III and stage II tumor disease is indicative that the test sample is from a patient suffering from stage II tumor disease; and the level of PSMA in the test sample that falls between the standard levels associated with stage I and stage II tumor disease is indicative that the test sample is from a patient suffering from stage I tumor disease. Those skilled in the art can understand that the levels of PSMA associated with stages I, II, III, and IV tumors vary depending on the type of tumor. The stage of a tumor may be determined by comparing the level of PSMA in the test sample with the corresponding standard level of PSMA for the particular tumor stage, or by comparing the level of PSMA in the test sample with the levels of PSMA in samples from individuals or populations having known disease stages. The values of the standard levels associated with tumor stages I, II, III, and IV may be determined through differential comparative analysis and validation of the levels of PSMA in test samples obtained from a large number of individuals suffering from the PSMA-mediated associated disease and a large number of healthy individuals.

According to an embodiment of the present disclosure, the test sample includes at least one of blood, saliva, sweat, tissues, cells, blood, serum, plasma, feces, and urine.

According to an embodiment of the present disclosure, the PSMA-mediated associated disease includes cancer.

According to an embodiment of the present disclosure, the cancer is at least one of prostate cancer, lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head and neck cancer.

In a fifteenth aspect of the present disclosure, the present disclosure provides a method for assessing the prognosis of a PSMA-mediated associated disease. According to an embodiment of the present disclosure, the method includes: detecting PSMA in a test sample using the antibody or antigen-binding fragment in the first aspect; and determining a level of PSMA in the test sample based on a detection result of PSMA. As described above, the level of PSMA has a significant impact on cancer. After treatment of an individual suffering from an associated disease, the prognosis of the disease can be effectively evaluated by monitoring the level of PSMA in tissues or excreta of the individual, such as peripheral blood or urine. For example, the prognosis may be evaluated by comparing the level of PSMA in a subject before and after treatment, or by comparing the level of PSMA in a treated subject with the level of PSMA in a normal individual or a diseased individual. The antibody or antigen-binding fragment in the first aspect of the present disclosure, or the antibody or antigen-binding fragment expressed by the nucleic acid molecule, expression vector, or recombinant cell, is each capable of effectively binding to human PSMA. Therefore, the method of the present disclosure is capable of efficiently detecting the level of PSMA in a test sample from a subject and assessing the prognosis of a PSMA-mediated associated disease based on the level of PSMA.

According to an embodiment of the present disclosure, the method for assessing the prognosis of the disease may further include at least one of the following additional technical features.

According to an embodiment of the present disclosure, the test sample is from a patient suffering from a PSMA-mediated associated disease before and after treatment.

According to an embodiment of the present disclosure, the test sample includes at least one of blood, saliva, sweat, tissues, cells, blood, serum, plasma, feces, and urine.

According to an embodiment of the present disclosure, the prognosis effect of the PSMA-mediated associated disease is determined based on the level of PSMA in the test sample of the patient suffering from the associated disease caused by PSMA before and after treatment.

According to an embodiment of the present disclosure, the PSMA-mediated associated disease includes cancer.

According to an embodiment of the present disclosure, the cancer is at least one of prostate cancer, lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head and neck cancer.

Amino acid sequences involved herein are shown in Table B.

**[Table B] Amino Acid Sequences**

| | Amino acid sequence | SEQ ID NO: |
|---|---|---|
| PSMAv11 HCDR1 | GYSFTHNW | 1 |
| PSMAv11/ parental mAb HCDR2 | IYPGDSDT | 2 |
| PSMAv11/ parental mAb HCDR3 | ARQTGFLWSSDLWGRGT | 3 |
| PSMAv11 LCDR1 | PQDISYA | 4 |
| PSMAv11 LCDR2 | DASW | 5 |
| PSMAv11 LCDR3 | QQFNSYPLS | 6 |
| PSMAv11 heavy chain variable region | EVQLVQSGAEVKKPGESLKISCKGSGYSFTHNWIGWVRQMPG KGLEWMGIIYPGDSDTRYSPSFQGQVTISADKSISTAYLQWSSL KASDTAMYYCARQTGFLWSSDLWGRGTLVTVSS | 7 |
| PSMAv11 light chain variable region | AIQLTQSPSSLSASVGDRVTITCRAPQDISYALAWYQQKPGKAP KLLIYDΛSWLESGVPSRFSGYGSGTDFTLTINSLQPEDFΛTYYC QQFNSYPLSFGGGTKVEIK | 8 |
| Heavy chain constant region | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSCDKTHTCPPCPAPELLGGPSVFLFPPKPKDT LMISRTPEVTCVVVDVSHEDPEVKFNWYVDGVEVHNAKTKPR EEQYNSTYRVVSVLTVLHQDWLNGKEYKCKVSNKALPAPIEKT ISKAKGQPREPQVYTLPPSREEMTKNQVSLTCLVKGFYPSDIAV EWESNGQPENNYKTTPPVLDSDGSFFLYSKLTVDKSRWQQGN VFSCSVMHEALHNHYTQKSLSLSPGK | 9 |
| Light chain constant region | | 10 |
| PSMAv11 heavy chain | EVQLVQSGAEVKKPGESLKISCKGSGYSFTHNWIGWVRQMPG KGLEWMGIIYPGDSDTRYSPSFQGQVTISADKSISTAYLQWSSL KASDTAMYYCARQTGFLWSSDLWGRGTLVTVSSASTKGPSVFP LAPSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPELLGGPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSREEMTKNQVSLTCLVKGFYPSDIAVEWESNGQPE NNYKTTPPVLDSDGSFFLYSKLTVDKSRWQQGNVFSCSVMHEA LHNHYTQKSLSLSPGK | 11 |
| PSMAv11 light chain/first peptide chain of the first binding region | AIQLTQSPSSLSASVGDRVTITCRAPQDISYALAWYQQKPGKAP KLLIYDASWLESGVPSRFSGYGSGTDFTLTINSLQPEDFATYYC QQFNSYPLSFGGGTKVEIKRTVAAPSVFIFPPSDEQLKSGTASVV CLLNNFYPREAKVQWKVDNALQSGNSQESVTEQDSKDSTYSL SSTLTLSKADYEKHKVYACEVTHQGLSSPVTKSFNRGEC | 12 |
| CD3 single-chain antibody | EVQLLESGGGLVQPGGSLKLSCAASGFTFNTYAMNWVRQAPG KGLEWVARIRSKYNNYATYYADSVKDRFTISRDDSKNTAYLQM NNLKTEDTAVYYCVRHGNFGNSYVSWFAYWGQGTLVTVSSGG GGSGGGGSGGGGSELVVTQEPSLTVSPGGTVTLTCRSSTGAVTT SNYANWVQQKPGQAPRGLIGGTNKRAPGTPARFSGSLLGGKA ALTLSGVQPEDEAEYYCALWYSNLWVFGGGTKLTVL | 13 |
| Linking peptide | GGGGS | 14 |
| First Fc fragment | PKSSDKTHTCPPCPAPEAAGGPSVFLFPPKPKDTLMISRTPEVTC VVVDVSHEDPEVKFNWYVDGVEVHNAKTKPREEQYNSTYRV VSVLTVLHQDWLNGKEYKCKVSNKALPAPIEKTISKAKGQPRE PQVYTLPPCREEMTKNQVSLWCLVKGFYPSDIAVEWESNGQPE NNYKTTPPVLDSDGSFFLYSKLTVDKSRWQQGNVFSCSVMHEA LHNHYTQKSLSLSP | 15 |
| Second Fc fragment | PKSCDKTHTCPPCPAPEAAGGPSVFLFPPKPKDTLMISRTPEVTC VVVDVSHEDPEVKFNWYVDGVEVHNAKTKPREEQYNSTYRV VSVLTVLHQDWLNGKEYKCKVSNKALPAPIEKTISKAKGQPRE PQVCTLPPSREEMTKNQVSLSCAVKGFYPSDIAVEWESNGQPE NNYKTTPPVLDSDGSFFLVSKLTVDKSRWQQGNVFSCSVMHEA LHNHYTQKSLSLSP | 16 |
| Second binding region (binding to CD3) | EVQLLESGGGLVQPGGSLKLSCAASGFTFNTYAMNWVRQAPG KGLEWVARIRSKYNNYATYYADSVKDRFTISRDDSKNTAYLQM NNLKTEDTAVYYCVRHGNFGNSYVSWFAYWGQGTLVTVSSGG GGSGGGGSGGGGSELVVTQEPSLTVSPGGTVTLTCRSSTGAVTT SNYANWVQQKPGQAPRGLIGGTNKRAPGTPARFSGSLLGGKA ALTLSGVQPEDEAEYYCALWYSNLWVFGGGTKLTVLGGGGSP KSSDKTHTCPPCPAPEAAGGPSVFLFPPKPKDTLMISRTPEVTCV VVDVSHEDPEVKFNWYVDGVEVHNAKTKPREEQYNSTYRVV SVLTVLHQDWLNGKEYKCKVSNKALPAPIEKTISKAKGQPREP QVYTLPPCREEMTKNQVSLWCLVKGFYPSDIAVEWESNGQPEN NYKTTPPVLDSDGSFFLYSKLTVDKSRWQQGNVFSCSVMHEAL HNHYTQKSLSLSP | 17 |
| First peptide chain of the first binding region (namely PSMAv11-hoss) | EVQLVQSGAEVKKPGESLKISCKGSGYSFTHNWIGWVRQMPG KGLEWMGIIYPGDSDTRYSPSFQGQVTISADKSISTAYLQWSSL KASDTAMYYCARQTGFLWSSDLWGRGTLVTVSSASTKGPSVFP LAPSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPEAAGGPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQPR EPQVCTLPPSREEMTKNQVSLSCAVKGFYPSDIAVEWESNGQPE NNYKTTPPVLDSDGSFFLVSKLTVDKSRWQQGNVFSCSVMHEA LHNHYTQKSLSLSP | 18 |
| parental mAb heavy chain | EVQLVQSGAEVKKPGESLKISCKGSGYSFTSNWIGWVRQMPG KGLEWMGIIYPGDSDTRYSPSFQGQVTISADKSISTAYLQWSSL KASDTAMYYCARQTGFLWSSDLWGRGTLVTVSSASTKGPSVFP LAPSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPELLGGPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSREEMTKNQVSLTCLVKGFYPSDIAVEWESNGQPE NNYKTTPPVLDSDGSFFLYSKLTVDKSRWQQGNVFSCSVMHEA LHNHYTQKSLSLSPGK | 19 |
| parental mAb light chain | AIQLTQSPSSLSASVGDRVTITCRASQDISSALAWYQQKPGKAP KLLIYDASSLESGVPSRFSGYGSGTDFTLTINSLQPEDFATYYCQ QFNSYPLTFGGGTKVEIKRTVAAPSVFIFPPSDEQLKSGTASVVC LLNNFYPREAKVQWKVDNALQSGNSQESVTEQDSKDSTYSLS STLTLSKADYEKHKVYACEVTHQGLSSPVTKSFNRGEC | 20 |
| First peptide chain of the first binding region (parental PSMA-hoss) | EVQLVQSGAEVKKPGESLKISCKGSGYSFTSNWIGWVRQMPG KGLEWMGIIYPGDSDTRYSPSFQGQVTISADKSISTAYLQWSSL KASDTAMYYCARQTGFLWSSDLWGRGTLVTVSSASTKGPSVFP LAPSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPEAAGGPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQPR EPQVCTLPPSREEMTKNQVSLSCAVKGFYPSDIAVEWESNGQPE NNYKTTPPVLDSDGSFFLVSKLTVDKSRWQQGNVFSCSVMHEA LHNHYTQKSLSLSP | 21 |
| CH1 | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVE | 22 |
| parental mAb heavy chain variable region | EVQLVQSGAEVKKPGESLKISCKGSGYSFTSNWIGWVRQMPG KGLEWMGIIYPGDSDTRYSPSFQGQVTISADKSISTAYLQWSSL KASDTAMYYCARQTGFLWSSDLWGRGTLVTVSS | 23 |
| parental mAb light chain variable region | AIQLTQSPSSLSASVGDRVTITCRASQDISSALAWYQQKPGKAP KLLIYDASSLESGVPSRFSGYGSGTDFTLTINSLQPEDFATYYCQ QFNSYPLTFGGGTKVEIK | 24 |

The solutions of the present disclosure will be illustrated with reference to examples. Those skilled in the art can understand that the following examples are only used to illustrate the present disclosure and should not be regarded as limitations of the scope of the present disclosure. If the specific technologies or conditions are not specified in the examples, they shall be carried out according to the technologies or conditions as described in the literature in the art or according to the product instructions. The reagents and instruments used without indicating the manufacturer are all conventional products that can be purchased commercially.

Unless otherwise indicated, the practice of the present disclosure will employ conventional techniques of cell biology, molecular biology (including recombinant technology), microbiology, biochemistry, and immunology, which are within the capabilities of those skilled in the art. Such techniques are fully explained in literatures, such as Molecular Cloning: A Laboratory Manual, second edition (Sambrook et al., 1989); Oligonucleotide Synthesis (edited by M.J. Gait, 1984); Animal Cell Culture (edited by R.I. Freshney, 1987); Methods in Enzymology (Academic Press, Inc.); Handbook of Experimental Immunology (edited by D. M. Weir and C. C. Blackwell); Gene Transfer Vectors for Mammalian Cells (edited by J. M. Miller and M. P. Calos, 1987); Current Protocols in Molecular Biology (edited by F. M. Ausubel et al., 1987); PCR: The Polymerase Chain Reaction (edited by Mullis et al., 1994); and Current Protocols in Immunology (edited by J. E. Coligan et al., 2011), each of which is expressly incorporated herein by reference.

In the embodiments of the present disclosure, the nucleotide sequences used to prepare the expression vectors can be obtained based on their amino acid sequences using conventional methods or conventional software (such as the online program Vectorbuilder (website: www.vectorbuilder.cn/tool/codon-optimization.html), GeneOptimizer online program, etc.).

### Example 1: Preparation of Antibody

The antibodies were prepared according to the following procedures:
(1) ExpiCHO cells (purchased from Thermo Fisher) were cultured in ExpiCHO Expression Medium (purchased from Thermo Fisher), and the cell concentration was adjusted as 6×10⁶/mL to obtain an ExpiCHO cell suspension.
(2) When the antibody was a monoclonal antibody, pcDNA3.4 vectors containing nucleotide sequences encoding the antibody heavy chain and antibody light chain (synthesized by GenScript, Nanjing) were added into 2 mL of OptiSFM medium (purchased from Thermo Fisher) according to a ratio of 1:1 to obtain Solution A.
   When the antibody was a bispecific antibody, pcDNA3.4 vectors containing nucleotide sequences encoding a CD3 antibody, a PSMA antibody heavy chain, and a PSMA antibody light chain (synthesized by GenScript, Nanjing) were added into 2 mL of OptiSFM medium (purchased from Thermo Fisher) according to a ratio of 1:1:1 to obtain Solution A.
(3) 160 µl of ExpiFectamineCHO transfection reagent (purchased from Thermo Fisher) was added into 2 mL of OptiSFM medium (purchased from Thermo Fisher) to obtain Solution B.
(4) Solution A and Solution B were then mixed to obtain a transfection mixture, and the entirely transfection mixture was added into 50 mL of the ExpiCHO cell suspension within 5 minutes.
(5) After culture at 37°C under 5% CO₂ conditions for 1 day, 8 mL of Feed and 300 µl of Enhancer (purchased from Thermo Fisher) were added, and the cells were further cultured at 32°C under 5% CO₂ conditions for 9 days before harvesting the culture supernatant. An additional 8 mL of Feed was added on Day 5.
(6) Affinity purification was performed on the cultural supernatant by using a Protein A purification column (purchased from Nano-Micro), thereby obtaining the target antibody.

### Example 2: Antibody Affinity Maturation

During the affinity maturation process of natural antibodies, somatic hypermutations are mainly concentrated in the CDR regions. By performing single-site saturation mutagenesis at each residue within the CDR regions *in vitro*, sufficient mutation diversity can be obtained without disrupting the protein structure. This approach enables the closest *in vitro* recapitulation of the *in vivo* somatic hypermutation process occurring in natural antibodies

Each amino acid residue within the CDR region was subjected to single-site saturation mutagenesis to construct an unbiased single-site saturation mutagenesis plasmid library of the parental antibody. Mutant sites exhibiting enhanced antigen-specific binding were screened by ELISA, and these sites were further combined and screened to obtain candidate antibody mutant sequences.

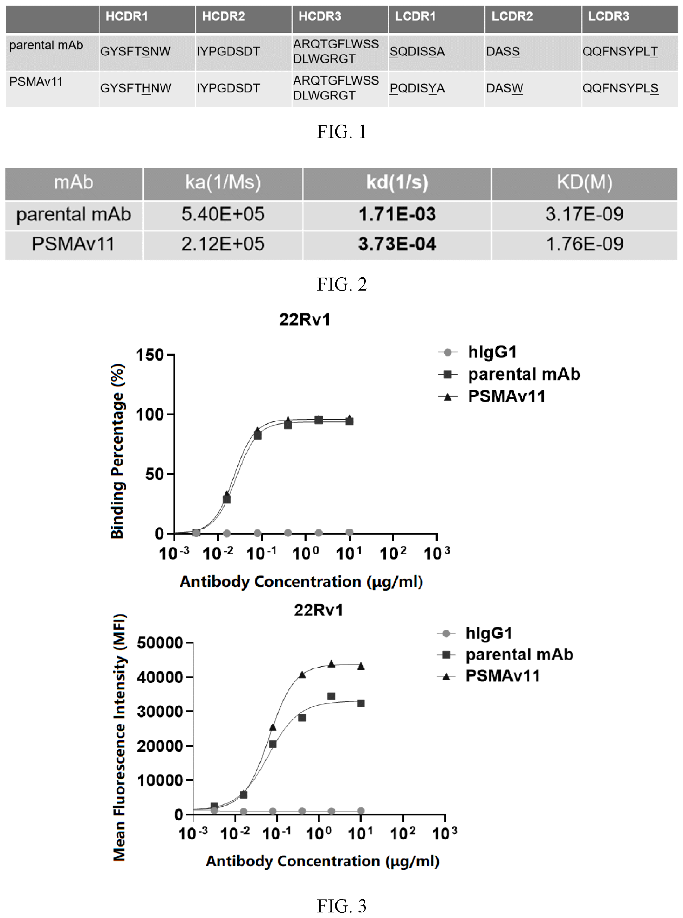

Through the above technical route, affinity optimization was performed on the parental PSMA antibody parental mAb (prepared by the method in Example 1, having a heavy chain variable region as set forth in SEQ ID NO: 23 and a light chain variable region as set forth in SEQ ID NO: 24, and the amino acid sequence of the heavy chain is as set forth in SEQ ID NO: 19 and the amino acid sequence of the light chain is as set forth in SEQ ID NO: 20), thereby obtaining a PSMA monoclonal antibody PSMAv11 exhibiting a slower dissociation rate (prepared by the method in Example 1). The amino acid sequence of the heavy chain of PSMAv11 is as set forth in SEQ ID NO: 11, and the amino acid sequence of the light chain thereof is as set forth in SEQ ID NO: 12. PSMAv11 has a heavy chain variable region with an amino acid sequence as set forth in SEQ ID NO: 7 and a light chain variable region with an amino acid sequence as set forth in SEQ ID NO: 8. Comparison of the CDR sequences between the parental antibody mAb and the affinity-matured antibody PSMAv11 is shown in FIG. 1.

Further, the parental mAb antibody and the PSMAv11 antibody prepared in this example were used in the following Examples 3 to 6.

### Example 3: Antibody Affinity Assay

Biacore is a method for analyzing biomolecular interactions based on the principle of surface plasmon resonance (SPR). It can not only detect specific binding between antigens and antibodies, but also provide highly important parameters for drug development, including the association rate constant (Ka), dissociation rate constant (Kd), and equilibrium dissociation constant (KD), thereby allowing calculation of antibody affinity.

In a Biacore 1K system (Cytiva), antibodies were diluted to 10 µg/mL with running buffer (HBS-EP), and immobilized onto a Protein A chip (Cytiva, 29127556) at a flow rate of 10 µL/min. Kinetic and affinity data for antigen-antibody binding were measured at a flow rate of 30 µL/min, with an association time of 120 s and a dissociation time of 800 s.

The kinetic and affinity data for the binding of the parental antibody parental mAb and the affinity-optimized antibody PSMAv11 to PSMA were determined. The results are shown in FIG. 2 that, compared with the parental antibody parental mAb, the affinity of the affinity-optimized antibody PSMAv11 for PSMA was improved by approximately 1.8-fold, while the dissociation rate was reduced by approximately 4.6-fold.

Notes: Ka represents the association rate constant, where a larger value indicates stronger affinity; Kd represents the dissociation rate constant, where a smaller value indicates stronger affinity, reflecting the affinity of the compound for the target; KD represents Kd/Ka, i.e., the equilibrium dissociation constant (affinity constant), where a smaller KD indicates less dissociation and thus stronger affinity.

### Example 4: ELISA Binding Assay for PSMA Antibody

ELISA was used to evaluate the binding characteristics of the parental antibody parental mAb and the affinity-optimized antibody PSMAv11 to PSMA. PSMA protein was coated onto a 96-well plate, and the signal intensity after antibody addition was used to determine the binding characteristics of the antibodies to PSMA.

PSMA protein (purchased from Acro) was diluted to 1 µg/mL with PBS buffer, added to a 96-well plate at 100 µL/well, and incubated overnight at 4°C. The PBS buffer was removed from the 96-well plate. The plate was washed 6 times with PBST buffer (that is, PBS having a pH value of 7.2 containing 0.1 volume% of Tween 20). Subsequently, 200 µL/well of PBS containing 10% of BSA was added and incubated at 37°C for 2 hours for blocking. After removal of the blocking solution and washing the plate six times with PBST, 100 µL/well of serially diluted parental antibody parental mAb, affinity-optimized antibody PSMAv11, and control IgG1 (purchased from Biointron) prepared in PBST containing 0.05% BSA were added. The highest working concentration was 20,000 ng/mL, followed by 5-fold serial dilutions over 8 gradients. The plate was incubated at 37°C for 1 h. The reaction system in the wells was removed. The plate was washed 6 times with PBST. Thereafter, 100 µL/well of HRP (horseradish peroxidase)-labeled anti-human secondary antibody (Fab-specific) (purchased from Sigma) diluted in PBST containing 0.05% BSA was added and incubated for 1 hour at 37°C. The secondary antibody in the wells was removed. The plate was washed 6 times with PBST. Subsequently, 80 µL/well of TMB (tetramethylbenzidine) was added and incubated at room temperature for 3 min, and then the reaction was terminated by adding 80 µL/well of 4 M sulfuric acid. Absorbance was measured at 450 nm using a microplate reader

The results indicated that the affinity-optimized antibody PSMAv11 of the present disclosure was capable of binding to PSMA protein.

### Example 5: Flow Cytometry Binding Assay for PSMA Antibody

Tumor cells (22RV1 or LNCaP human prostate cancer cells) were diluted to 2×10⁶ cells /mL with PBS and added to 1.5 mL EP tubes at 100 µL/tube. Goat serum was added at 10 µL/tube for blocking at 4°C for 30 minutes. Serially diluted parental antibody parental mAb, affinity-optimized antibody PSMAv11, and control IgG1 (purchased from Biointron) were added. The highest working concentration was 50 µg/mL, followed by 5-fold serial dilutions over 10 gradients. The mixture was incubated at 4°C for 30 min. Subsequently, 1 mL of PBS was added to each EP tube, followed by centrifugation at 3500 rpm at 4°C for 5 minutes. The supernatant was completely discarded, and the cells were washed once with PBS. After centrifugation, the supernatant was completely discarded, and the cells were resuspended in 100 µL/tube of PBS. Subsequently, 1 µL/tube of Alexa-647-labeled goat anti-human secondary antibody (purchased from Jackson lab) was added and incubated at 4°C in the dark for 30 minutes. The cells were washed twice with PBS and the supernatant was discarded completely after centrifugation. The cells were resuspended in 200 µL/tube of PBS and analyzed by flow cytometry.

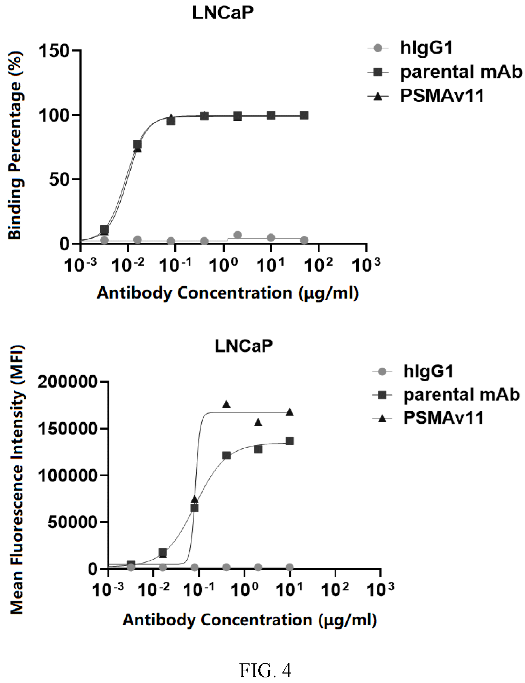

The results are shown in FIG. 3 and FIG. 4 that the affinity-optimized antibody PSMAv11 of the present disclosure exhibited higher fluorescence intensity upon binding to 22RV1 and LNCaP human prostate cancer cells than the parental antibody, indicating that the affinity-optimized antibody PSMAv11 possessed higher binding activity.

### Example 6: PSMA Antibody-Promoted PBMC-Mediated Tumor Cell Killing

The ability of the PSMA monoclonal antibody to promote PBMC-mediated killing of 22RV1 and LNCaP human prostate cancer cells was evaluated.
(1) Complete RPMI-1640 medium was added into a 16-well RTCA plate at 50 µL/well for instrument calibration.
(2) Tumor cells were diluted to 2×10⁵ cells/mL with complete RPMI-1640 medium and separately added to the RTCA plate obtained in step (1) at 50 µl/well. The cell index was then monitored for 24 hours at 37°C under 5% CO₂ conditions using an xCELLigence RTCA MP instrument.
(3) The parental antibody parental mAb and affinity-optimized antibody PSMAv11 were serially diluted with complete RPMI-1640 medium and added to the RTCA plate obtained in step (2) at 20 µL/well.
(4) PBMCs (purchased from Saily Biotechnology) were diluted to 1.25×10⁶ cells/mL with complete RPMI-1640 medium and added to the RTCA plate obtained in step (3) at 80 µl/well.
(5) The reaction system obtained in step (4) was monitored for cell index for 24 hours at 37°C under 5% CO₂ conditions using an xCELLigence RTCA MP instrument.

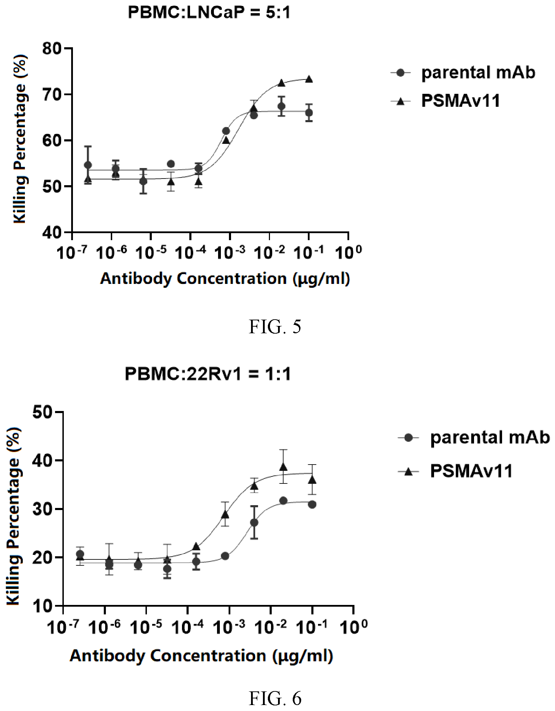

As shown in FIG. 5 and FIG. 6, the PSMAv11 antibody of the present disclosure promoted PBMC-mediated tumor cell killing more effectively than the parental antibody parental mAb.

### Example 7: Flow Cytometry Binding Assay for CD3×PSMA Antibody

An experimental group CD3×PSMAv11 bispecific antibody and a control CD3×parental mAb bispecific antibody were prepared according to the method described in Example 1. For the CD3×PSMAv11 bispecific antibody, the amino acid sequence of the CD3 antibody is as set forth in SEQ ID NO: 17, the amino acid sequence of the heavy chain is as set forth in SEQ ID NO: 18, and the amino acid sequence of the light chain is as set forth in SEQ ID NO: 12. For the CD3×parental mAb bispecific antibody, the amino acid sequence of the CD3 antibody is as set forth in SEQ ID NO: 17, the amino acid sequence of the heavy chain is as set forth in SEQ ID NO: 21, and the amino acid sequence of the light chain is as set forth in SEQ ID NO: 20. Flow cytometry binding assay was then performed, and the specific experimental procedures were as follows:

Tumor cells were diluted to 2×10⁶ cells/mL with PBS and added to a 1.5 mL EP tube at 100 µL/tube. Goat serum was added at 10 µL/tube for blocking at 4°C for 30 minutes. Serially diluted CD3×parental PSMA bispecific antibody, CD3×PSMAv11 bispecific antibody, and control IgG1LALA (purchased from Biointron) were added and incubated at 4°C for 30 minutes. Subsequently, 1 mL PBS was added to each EP tube, followed by centrifugation at 3,500 rpm at 4°C for 5 minutes. The supernatant was completely discarded and the cells were washed once with PBS. After centrifugation, the supernatant was completely discarded, and the cells were resuspended in 100 µL/tube of PBS. Subsequently, 1 µL/tube of Alexa-647-labeled goat anti-human secondary antibody (purchased from Jackson lab) was added and incubated at 4°C in the dark for 30 minutes. The cells were washed twice with PBS and the supernatant was discarded after centrifugation. The cells were resuspended in 200 µL/tube of PBS and analyzed by flow cytometry.

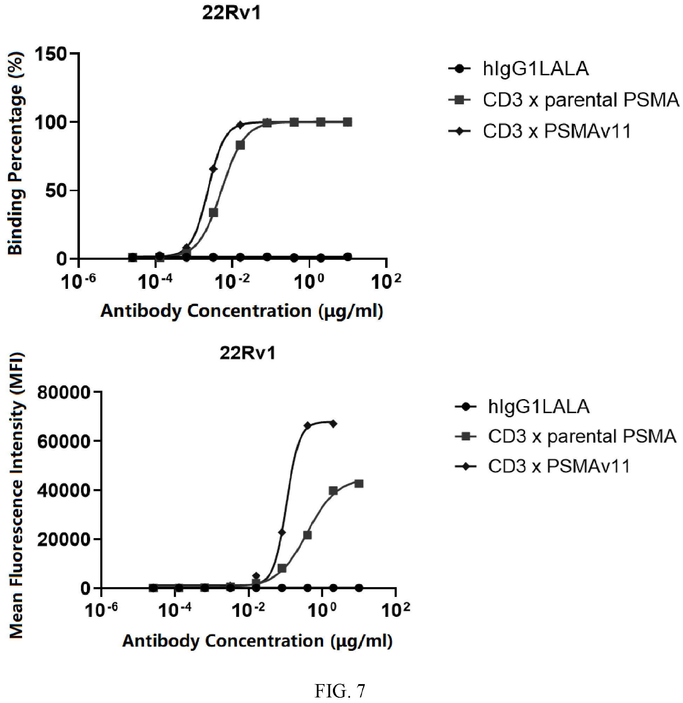

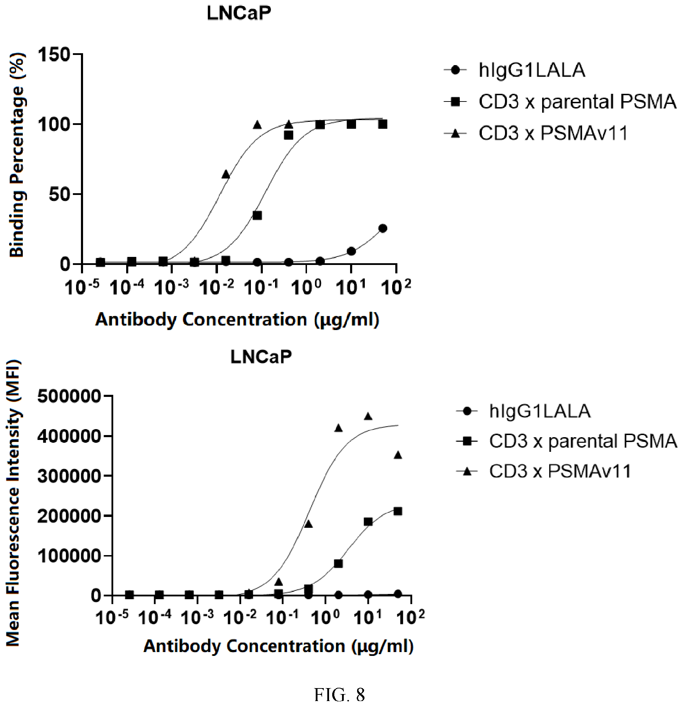

The results are shown in FIG. 7 and FIG. 8 that CD3×PSMAv11 of the present disclosure exhibited stronger binding to 22RV1 and LNCaP human prostate cancer cells than the CD3×parental PSMA bispecific antibody

### Example 8: CD3×PSMA Antibody-Promoted PBMC-Mediated Tumor Cell Killing

The ability of the CD3×PSMA bispecific antibody and the CD3×parental mAb bispecific antibody obtained in Example 7 to promote PBMC-mediated killing of 22RV1 and LNCaP human prostate cancer cells was evaluated. The specific procedures were as follows:
(1) Complete RPMI-1640 medium was added into a 16-well RTCA plate at 50 µL/well for instrument calibration.
(2) Tumor cells were diluted to 2×10⁵ cells/mL with complete RPMI-1640 medium and separately added to the RTCA plate obtained in step (1) at 50 µl/well. The cell index was then monitored for 24 hours at 37°C under 5% CO₂ conditions using an xCELLigence RTCA MP instrument.
(3) The CD3×parental PSMA bispecific antibody, the CD3×PSMAv11 bispecific antibody, and control IgG1LALA (purchased from Biointron) were serially diluted with complete RPMI-1640 medium and added to the RTCA plate obtained in step (2) at 20 µL/well.
(4) PBMCs (purchased from Saily Biotechnology) were diluted to 1.25×10⁶ cells/mL with complete RPMI-1640 medium and added to the RTCA plate obtained in step (3) at 80 µl/well.
(5) The reaction system obtained in step (4) was monitored for cell index for 24 hours at 37°C under 5% CO₂ conditions using an xCELLigence RTCA MP instrument.

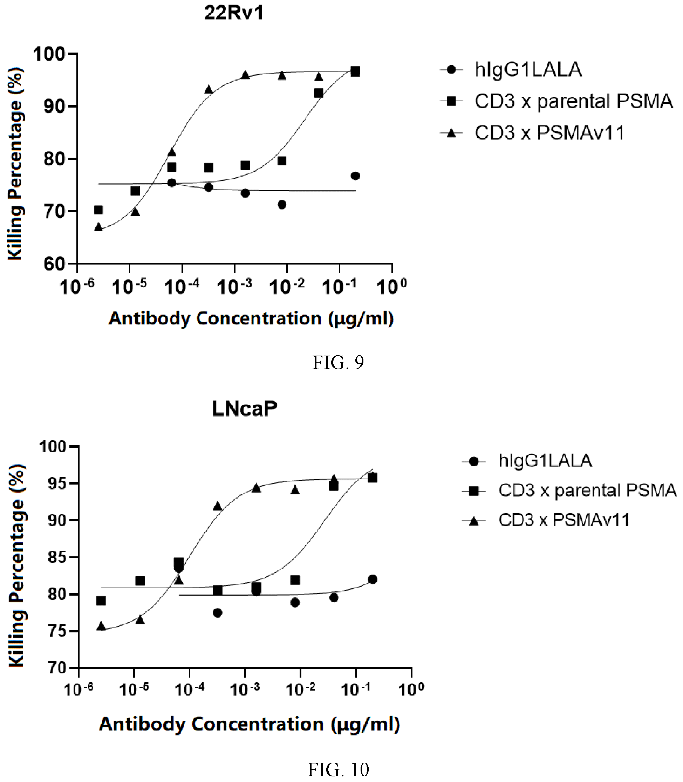

As shown in FIG. 9 and FIG. 10, the CD3×PSMAv11 antibody of the present disclosure promoted PBMC-mediated tumor cell killing far more effectively than the CD3×parental PSMA bispecific antibody

Reference throughout this specification to terms such as "an embodiment", "some embodiments", "an example", "a specific example", or "some examples" means that a particular feature, structure, material, or characteristic described in connection with the embodiment or example is included in at least one embodiment or example of the present disclosure. The appearances of the above phrases in various places throughout this specification are not necessarily referring to the same embodiment or example. Further, the particular features, structures, materials, or characteristics may be combined in any suitable manner in one or more embodiments or examples. In addition, different embodiments or examples and features of different embodiments or examples described in the specification may be combined by those skilled in the art without mutual contradiction.

Although embodiments of the present disclosure have been shown and described above, it should be understood that above embodiments are merely exemplary, and cannot be construed to limit the present disclosure. For those skilled in the art, changes, alternatives, and modifications can be made to the embodiments without departing from the scope of the present disclosure.

## Claims

1. An antibody or antigen-binding fragment, comprising a CDR selected from at least one of:
HCDR 1: GYSFTX₁NW, wherein X₁ is S or H;
HCDR 2: IYPGDSDT;
HCDR 3: ARQTGFLWSSDLWGRGT;
LCDR 1: X₂QDISX₃A, wherein X₂ is S or P, and X₃ is S or Y;
LCDR 2: DASX₄, wherein X₄ is S or W; or
LCDR 3: QQFNSYPLX₅, wherein X₅ is T or S,
provided that X₁ being S, X₂ being S, X₃ being S, X₄ being S, and X₅ being T do not occur simultaneously.

2. The antibody or antigen-binding fragment according to claim 1, wherein X₁ is S, optionally, X₁ is H,
optionally, X₂ is S,
optionally, X₂ is P,
optionally, X₃ is S,
optionally, X₃ is Y,
optionally, X₄ is S,
optionally, X₄ is W,
optionally, X₅ is T,
optionally, X₅ is S,
optionally, the antibody or antigen-binding fragment comprises:
HCDR1, HCDR2, and HCDR3 with an amino acid sequence as set forth in SEQ ID NO: 1, SEQ ID NO: 2, and SEQ ID NO: 3, respectively; and
LCDR1, LCDR2, and LCDR3 with an amino acid sequence as set forth in SEQ ID NO: 4, SEQ ID NO: 5, and SEQ ID NO: 6, respectively,
optionally, the antibody or antigen-binding fragment thereof specifically recognizes PSMA.

3. The antibody or antigen-binding fragment according to claim 2, comprising a heavy chain framework region and/or a light chain framework region,
optionally, at least a portion of the heavy chain framework region and/or at least a portion of the light chain framework region is from at least one of a murine antibody, a primate antibody, a bovine antibody, an equine antibody, a dairy cow antibody, a porcine antibody, an ovine antibody, a caprine antibody, a canine antibody, a feline antibody, a rabbit antibody, a camelid antibody, a donkey antibody, a deer antibody, a mink antibody, a chicken antibody, a duck antibody, a goose antibody, a turkey antibody, a gamecock antibody, or a mutant thereof,
optionally, at least a portion of the heavy chain framework region and/or at least a portion of the light chain framework region is from at least one of a murine antibody and a human antibody;
optionally, the antibody or antigen-binding fragment comprises:
a heavy chain variable region with an amino acid sequence as set forth in SEQ ID NO: 7 or an amino acid sequence having at least 90% homology thereto; and
a light chain variable region with an amino acid sequence as set forth in SEQ ID NO:
8 or an amino acid sequence having at least 90% homology thereto.

4. The antibody or antigen-binding fragment according to any one of claims 1 to 3, further comprising a heavy chain constant region and/or a light chain constant region,
optionally, at least a portion of at least one of the heavy chain constant region and the light chain constant region is from at least one of a murine antibody, a primate antibody, a bovine antibody, an equine antibody, a dairy cow antibody, a porcine antibody, an ovine antibody, a caprine antibody, a canine antibody, a feline antibody, a rabbit antibody, a camelid antibody, a donkey antibody, a deer antibody, a mink antibody, a chicken antibody, a duck antibody, a goose antibody, a turkey antibody, a gamecock antibody, or a mutant thereof,
optionally, the heavy chain constant region comprises a heavy chain constant region selected from IgG1, IgG2, IgG3, IgG4, IgA, IgM, IgE, or IgD, or the light chain constant region comprises a light chain constant region selected from a κ-type light chain constant region or a λ-type light chain constant region,
optionally, the light chain constant region and the heavy chain constant region are both from a murine antibody or a mutant thereof, or a human antibody or a mutant thereof,
optionally, the N-terminus of the heavy chain constant region is linked to the C-terminus of the heavy chain variable region, and/or the N-terminus of the light chain constant region is linked to the C-terminus of the light chain variable region,
optionally, the heavy chain constant region comprises a heavy chain constant region with an amino acid sequence as set forth in SEQ ID NO: 9 or an amino acid sequence having at least 80% identity thereto; and/or the light chain constant region comprises a light chain constant region with an amino acid sequence as set forth in SEQ ID NO: 10 or an amino acid sequence having at least 80% identity thereto,
optionally, the antibody comprises at least one of a polyclonal antibody, a full-length monoclonal antibody, a Fab antibody, a Fab' antibody, a F(ab')₂ antibody, a Fv antibody, a single-chain antibody, a single-domain antibody, or a minimum recognition unit; or the antigen-binding fragment comprises at least one of a F(ab')₂ fragment, a Fab' fragment, a Fab fragment, a F(ab)₂ fragment, a Fv fragment, a scFv fragment, a scFv-Fc fusion protein, a scFv-Fv fusion protein, or a minimum recognition unit,
optionally, the antibody or antigen-binding fragment comprises:
a heavy chain with an amino acid sequence as set forth in SEQ ID NO: 11 or an amino acid sequence having at least 80% homology thereto; and
a light chain with an amino acid sequence as set forth in SEQ ID NO: 12 or an amino acid sequence having at least 80% homology thereto.

5. A multispecific antibody, comprising:
a first binding region, comprising the antibody or antigen-binding fragment according to any one of claims 1 to 4; and
a second binding region, having a binding activity to a first molecule.

6. The multispecific antibody according to claim 5, wherein the first molecule is selected from at least one of an immune cell surface antigen, a tumor antigen, a virus, a bacterium, an endotoxin, a cytokine, or a cytokine receptor;
optionally, the first molecule is selected from at least one of CD3, PD-L1, PD-1, IL-10, IL-10R, BCMA, VEGF, TGF-β, CTLA-4, LAG-3, TIGIT, CEA, CD38, SLAMF7, B7-H3, Her2, EpCAM, CD19, CD20, CD30, CD33, CD47, CD52, CD133, EGFR, GD2, CD3, GM2, RANKL, or CD16a;
optionally, the second binding region is a binding protein or fragment thereof of the first molecule;
optionally, the second binding region is at least one of an antibody or antigen-binding fragment of the first molecule and a receptor fragment of the first molecule;
optionally, the second binding region is a single-chain antibody of the first molecule; optionally, the second binding region further comprises a first Fc fragment;
optionally, the C-terminus of the binding protein or fragment thereof of the first molecule is linked to the N-terminus of the first Fc fragment;
optionally, the second binding region further comprises a linking peptide;
optionally, the C-terminus of the binding protein or fragment thereof of the first molecule is linked to the N-terminus of the linking peptide, and the C-terminus of the linking peptide is linked to the N-terminus of the first Fc fragment;
optionally, the linking peptide has an amino acid sequence as set forth in (GGGGS)ₙ, wherein n is an integer greater than or equal to 1, preferably 1, 2, 3, 4, 5, 6, 7, 8, 9, or 10;
optionally, the bispecific antibody comprises a symmetric bispecific antibody or an asymmetric bispecific antibody, and preferably, the bispecific antibody is an asymmetric bispecific antibody;
optionally, the first molecule is CD3, and the second binding region comprises an anti-CD3 antibody;
optionally, the anti-CD3 antibody is selected from at least one of a Fab antibody, a Fab' antibody, a F(ab')₂ antibody, a Fv antibody, a single-chain antibody, a single-domain antibody, or a minimum recognition unit;
optionally, the anti-CD3 antibody is a CD3 single-chain antibody;
optionally, the C-terminus of the CD3 single-chain antibody is linked to the N-terminus of the linking peptide, and the C-terminus of the linking peptide is linked to the N-terminus of the first Fc fragment;
optionally, the CD3 single-chain antibody has an amino acid sequence as set forth in SEQ ID NO: 13;
optionally, the linking peptide has an amino acid sequence as set forth in SEQ ID NO: 14;
optionally, the first Fc fragment has an amino acid sequence as set forth in SEQ ID NO: 15;
optionally, the first binding region has an amino acid sequence as set forth in SEQ ID NO: 17.

7. The multispecific antibody according to any one of claims 5 and 6, wherein the antibody or antigen-binding fragment in the first binding region is selected from at least one of a Fab antibody, a Fab' antibody, an F(ab')₂ antibody, a Fv antibody, a single-chain antibody, a single-domain antibody, or a minimum recognition unit, and preferably is a Fab antibody or a single-chain antibody,
optionally, the antibody or antigen-binding fragment in the first binding region is a Fab antibody,
optionally, the Fab antibody comprises CDRs in the antibody or antigen-binding fragment according to any one of claims 1 to 4,
optionally, the Fab antibody comprises a heavy chain variable region and a light chain variable region in the antibody or antigen-binding fragment according to any one of claims 1 to 4,
optionally, the first binding region further comprises a second Fc fragment, optionally, the C-terminus of CH1 in the Fab antibody is linked to the N-terminus of the second Fc fragment,
optionally, the second Fc fragment has an amino acid sequence as set forth in SEQ ID NO: 16,
optionally, the first binding region comprises:
a first peptide chain having an amino acid sequence as set forth in SEQ ID NO: 18; and
a second peptide chain having an amino acid sequence as set forth in SEQ ID NO: 12.

8. A nucleic acid molecule, encoding the antibody or antigen-binding fragment according to any one of claims 1 to 4, or the multispecific antibody according to any one of claims 5 to 7,
optionally, the nucleic acid molecule is DNA.

9. An expression vector, carrying the nucleic acid molecule according to claim 8,
optionally, the expression vector is selected from a eukaryotic expression vector or a prokaryotic expression vector.

10. A recombinant cell, wherein:
the recombinant cell carries the nucleic acid molecule according to claim 8 or the expression vector according to claim 9; or
the recombinant cell expresses the antibody or antigen-binding fragment according to any one of claims 1 to 4, or the multispecific antibody according to any one of claims 5 to 7,
optionally, the recombinant cell is obtained by introducing the expression vector according to claim 9 into a host cell.

11. A conjugate, comprising:
the antibody or antigen-binding fragment according to any one of claims 1 to 4 or the multispecific antibody according to any one of claims 5 to 7; and
a conjugate moiety, linked to the antibody or antigen-binding fragment or the multispecific antibody,
optionally, the conjugate moiety comprises at least one selected from a carrier, a drug, a toxin, a cytokine, a protein tag, or a modifier.

12. A pharmaceutical composition, comprising:
the antibody or antigen-binding fragment according to any one of claims 1 to 4, the multispecific antibody according to any one of claims 5 to 7, the nucleic acid molecule according to claim 8, the expression vector according to claim 9, the recombinant cell according to claim 10, or the conjugate according to claim 11; and
optionally, a pharmaceutically acceptable excipient.

13. A kit, comprising
the antibody or antigen-binding fragment according to any one of claims 1 to 4, the multispecific antibody according to any one of claims 5 to 7, the nucleic acid molecule according to claim 8, the expression vector according to claim 9, the recombinant cell according to claim 10, or the conjugate according to claim 11.

14. The antibody or antigen-binding fragment according to any one of claims 1 to 4, the multispecific antibody according to any one of claims 5 to 7, the nucleic acid molecule according to claim 8, the expression vector according to claim 9, the recombinant cell according to claim 10, the conjugate according to claim 11, or the pharmaceutical composition according to claim 12 for use in the prevention and/or treatment of a PSMA-mediated associated disease, or use of the antibody or antigen-binding fragment according to any one of claims 1 to 4, the multispecific antibody according to any one of claims 5 to 7, the nucleic acid molecule according to claim 8, the expression vector according to claim 9, the recombinant cell according to claim 10, the conjugate according to claim 11, or the pharmaceutical composition according to claim 12 in:
the prevention and/or treatment of a PSMA-mediated associated disease; and/or
the manufacture of a medicament for preventing and/or treating a PSMA-mediated associated disease,
optionally, the PSMA-mediated associated disease comprises a tumor and/or cancer,
optionally, the cancer is prostate cancer.

15. The antibody or antigen-binding fragment according to any one of claims 1 to 4, the multispecific antibody according to any one of claims 5 to 7, the nucleic acid molecule according to claim 8, the expression vector according to claim 9, the recombinant cell according to claim 10, the conjugate according to claim 11, or the kit according to claim 13 for use in the detection of PSMA, in the detection of a PSMA-mediated associated disease, in the diagnosis of a PSMA-mediated associated disease, in the staging of a PSMA-mediated associated disease, or in the assessment of the prognosis of a PSMA-mediated associated disease, or use of the antibody or antigen-binding fragment according to any one of claims 1 to 4, the multispecific antibody according to any one of claims 5 to 7, the nucleic acid molecule according to claim 8, the expression vector according to claim 9, the recombinant cell according to claim 10, the conjugate according to claim 11, or the kit according to claim 13 in:
the detection of PSMA; and/or
the detection of a PSMA-mediated associated disease; and/or
the diagnosis of a PSMA-mediated associated disease; and/or
the staging of a PSMA-mediated associated disease; and/or
the assessment of the prognosis of a PSMA-mediated associated disease; and/or
the manufacture of a kit for detecting PSMA; and/or
in the manufacture of a kit for detecting a PSMA-mediated associated disease; and/or
in the manufacture of a kit for diagnosing a PSMA-mediated associated disease; and/or
in the manufacture of a kit for staging a PSMA-mediated associated disease; and/or
in the manufacture of a kit for assessing the prognosis of a PSMA-mediated associated disease,
optionally, the PSMA-mediated associated disease comprises a tumor and/or cancer,
optionally, the cancer is prostate cancer.

16. A method for preventing and/or treating a PSMA-mediated associated disease, the method comprising:
administering to a subject a pharmaceutically acceptable amount of the antibody or antigen-binding fragment according to any one of claims 1 to 4, the multispecific antibody according to any one of claims 5 to 7, the conjugate according to claim 11, or the pharmaceutical composition according to claim 12;
optionally, the PSMA-mediated associated disease comprises a tumor and/or cancer;
optionally, the cancer is prostate cancer.

17. A method for detecting PSMA, the method comprising:
bring a test sample into contact with the antibody or antigen-binding fragment according to any one of claims 1 to 4, the multispecific antibody according to any one of claims 5 to 7, the conjugate according to claim 11, or the kit according to claim 13 to form an immune complex.

18. A method for diagnosing a PSMA-mediated associated disease, staging a PSMA-mediated associated disease, or assessing the prognosis of a PSMA-mediated associated disease, the method comprising:
detecting PSMA in a test sample using the antibody or antigen-binding fragment according to any one of claims 1 to 4, the multispecific antibody according to any one of claims 5 to 7, or the conjugate according to claim 11; and
determining a level of PSMA in the test sample based on a detection result of PSMA.
